# EUROPEAN PATENT APPLICATION

(11) **EP 3 189 850 A1**
(43) Date of publication of application: **12.07.2017**
(21) Application number: 15200568.2
(22) Date of filing: 16.12.2015
(51) Int. Cl.: A61K 38/12, C07K 7/50, C07K 7/64, C07K 14/005, A61K 51/08, A61K 47/50, A61K 51/04

(54) **LIVER TARGETING OF CYCLIC PRES-DERIVED PEPTIDES OF HBV**

(71) Applicant: Ruprecht-Karls-Universität Heidelberg, 69117 Heidelberg (DE)
(72) Inventor: URBAN, Stephan, 67434 Neustadt/Weinstrasse (DE); NI, Yi, 69214 Eppelheim (DE); MIER, Walter, 64625 Bensheim (DE); KAUFMANN, Christina, 69117 Heidelberg (DE); MEHRLE, Stefan, 67117 Limburgerhof (DE); LINDNER, Thomas, 69214 Eppelheim (DE)
(74) Representative: Grahn, Sibylla Maria

(57) **Abstract**

The present invention relates to cyclic preS-derived peptides of HBV which are versatile vehicles for the targeted delivery of compounds to the liver, preferably to hepatocytes, *in vitro* as well as *in vivo.* The present invention further relates to conformationally constrained cyclic preS-derived peptides of HBV which are versatile vehicles for the targeted delivery of compounds to the liver, preferably to hepatocytes, *in vitro* as well as *in vivo.*

The present invention further relates to pharmaceutical compositions comprising at least one cyclic peptide. The present invention further relates to medical uses of said cyclic peptides and the pharmaceutical compositions, such as in the diagnosis, prevention and/or treatment of a liver disease or condition. The present invention further relates to methods for the specific delivery of compound(s) to the liver, methods for the diagnosis, prevention and/or treatment of a liver disease or condition and/or methods for the diagnosis, prevention and/or treatment of a cardiovascular disease.

SEQUENCE LISTING

<110>  Ruprecht-Karls-Universität Heidelberg

<120>  Liver targeting of cyclic preS-derived peptides of HBV

<130>  U30633EP

<160>  51

<170>  PatentIn version 3.5

<210>  1
<211>  7
<212>  PRT
<213>  Artificial Sequence

<220>
<223>  Essential domain or pharmacophore, amino acid sequence P, with
       Xaa = F or L

<220>
<221>  misc_feature
<222>  (6)..(6)
<223>  Xaa can be any naturally occurring amino acid

<400>  1

Asn Pro Leu Gly Phe Xaa Pro
1               5

<210>  2
<211>  7
<212>  PRT
<213>  Hepatitis B virus

<400>  2

Asn Pro Leu Gly Phe Phe Pro
1               5

<210>  3
<211>  7
<212>  PRT
<213>  Artificial Sequence

<220>
<223>  Essential domain or pharmacophore, amino acid sequence P with Xaa
       = L

<400>  3

Asn Pro Leu Gly Phe Leu Pro
1               5

<210>  4
<211>  9
<212>  PRT
<213>  Artificial Sequence

<220>
<223>  Cys-preS9-15-Cys

<400>  4

Cys Asn Pro Leu Gly Phe Phe Pro Cys
1               5

<210>  5
<211>  10
<212>  PRT
<213>  Artificial Sequence

<220>
<223>  Cys-HBVpreS9-15-Cys-D-Tyr

<400>  5

Cys Asn Pro Leu Gly Phe Phe Pro Cys Tyr
1               5                   10

<210>  6
<211>  8
<212>  PRT
<213>  Hepatitis B virus

<400>  6

Asn Pro Leu Gly Phe Phe Pro Asp
1               5

<210>  7
<211>  10
<212>  PRT
<213>  Artificial Sequence

<220>
<223>  Cys-HBVpreS9-16-Cys

<400>  7

Cys Asn Pro Leu Gly Phe Phe Pro Asp Cys
1               5                   10

<210>  8
<211>  11
<212>  PRT
<213>  Artificial Sequence

<220>
<223>  Cys-HBVpreS9-16-Cys-D-Tyr

<400>  8

Cys Asn Pro Leu Gly Phe Phe Pro Asp Cys Tyr
1               5                   10

<210>  9
<211>  9
<212>  PRT
<213>  Hepatitis B virus

<400>  9

Pro Asn Pro Leu Gly Phe Phe Pro Asp
1               5


<210>  10
<211>  11
<212>  PRT
<213>  Artificial Sequence

<220>
<223>  Cys-HBVpreS8-16-Cys

<400>  10

Cys Pro Asn Pro Leu Gly Phe Phe Pro Asp Cys
1               5                   10


<210>  11
<211>  12
<212>  PRT
<213>  Artificial Sequence

<220>
<223>  Cys-HBVpreS8-16-Cys-D-Tyr

<400>  11

Cys Pro Asn Pro Leu Gly Phe Phe Pro Asp Cys Tyr
1               5                   10


<210>  12
<211>  9
<212>  PRT
<213>  Hepatitis B virus

<400>  12

Asn Pro Leu Gly Phe Phe Pro Asp His
1               5


<210>  13
<211>  11
<212>  PRT
<213>  Artificial Sequence

<220>
<223>  Cys-HBVpreS9-17-Cys

<400>  13

Cys Asn Pro Leu Gly Phe Phe Pro Asp His Cys
1               5                   10

<210>  14
<211>  12
<212>  PRT
<213>  Artificial Sequence

<220>
<223>  Cys-HBVpreS9-17-Cys-D-Tyr

<400>  14

Cys Asn Pro Leu Gly Phe Phe Pro Asp His Cys Tyr
1               5                   10


<210>  15
<211>  10
<212>  PRT
<213>  Hepatitis B virus

<400>  15

Pro Asn Pro Leu Gly Phe Phe Pro Asp His
1               5                   10


<210>  16
<211>  12
<212>  PRT
<213>  Artificial Sequence

<220>
<223>  Cys-HBVpreS8-17-Cys

<400>  16

Cys Pro Asn Pro Leu Gly Phe Phe Pro Asp His Cys
1               5                   10


<210>  17
<211>  13
<212>  PRT
<213>  Artificial Sequence

<220>
<223>  Cys-HBVpreS8-17-Cys-D-Tyr

<400>  17

Cys Pro Asn Pro Leu Gly Phe Phe Pro Asp His Cys Tyr
1               5                   10


<210>  18
<211>  20
<212>  PRT
<213>  Hepatitis B virus

<400>  18

Gly Thr Asn Leu Ser Val Pro Asn Pro Leu Gly Phe Phe Pro Asp His
1               5                   10                  15

Gln Leu Asp Pro
            20

<210>  19
<211>  22
<212>  PRT
<213>  Artificial Sequence

<220>
<223>  Cys-HBVpreS2-21-Cys

<400>  19

Cys Gly Thr Asn Leu Ser Val Pro Asn Pro Leu Gly Phe Phe Pro Asp
1               5                   10                  15

His Gln Leu Asp Pro Cys
            20

<210>  20
<211>  23
<212>  PRT
<213>  Artificial Sequence

<220>
<223>  Cys-HBVpreS2-21-Cys-D-Tyr

<400>  20

Cys Gly Thr Asn Leu Ser Val Pro Asn Pro Leu Gly Phe Phe Pro Asp
1               5                   10                  15

His Gln Leu Asp Pro Cys Tyr
            20

<210>  21
<211>  47
<212>  PRT
<213>  Hepatitis B virus

<400>  21

Gly Thr Asn Leu Ser Val Pro Asn Pro Leu Gly Phe Phe Pro Asp His
1               5                   10                  15

Gln Leu Asp Pro Ala Phe Gly Ala Asn Ser Asn Asn Pro Asp Trp Asp
            20                  25                  30

Phe Asn Pro Asn Lys Asp His Trp Pro Glu Ala Asn Gln Val Gly
        35                  40                  45

<210>  22
<211>  49

<212>  PRT
<213>  Artificial Sequence

<220>
<223>  Cys-HBVpreS2-48-Cys

<400>  22

Cys Gly Thr Asn Leu Ser Val Pro Asn Pro Leu Gly Phe Phe Pro Asp
1               5                   10                  15

His Gln Leu Asp Pro Ala Phe Gly Ala Asn Ser Asn Asn Pro Asp Trp
            20                  25                  30

Asp Phe Asn Pro Asn Lys Asp His Trp Pro Glu Ala Asn Gln Val Gly
        35                  40                  45

Cys

<210>  23
<211>  50
<212>  PRT
<213>  Artificial Sequence

<220>
<223>  Cys-HBVpreS2-48-Cys-D-Tyr

<400>  23

Cys Gly Thr Asn Leu Ser Val Pro Asn Pro Leu Gly Phe Phe Pro Asp
1               5                   10                  15

His Gln Leu Asp Pro Ala Phe Gly Ala Asn Ser Asn Asn Pro Asp Trp
            20                  25                  30

Asp Phe Asn Pro Asn Lys Asp His Trp Pro Glu Ala Asn Gln Val Gly
        35                  40                  45

Cys Tyr
    50

<210>  24
<211>  47
<212>  PRT
<213>  Artificial Sequence

<220>
<223>  amino acid residues of Myrcludex B

<400>  24

Gly Thr Asn Leu Ser Val Pro Asn Pro Leu Gly Phe Phe Pro Asp His
1               5                   10                  15

Gln Leu Asp Pro Ala Phe Gly Ala Asn Ser Asn Asn Pro Asp Trp Asp
            20                  25                  30

Phe Asn Pro Asn Lys Asp His Trp Pro Glu Ala Asn Lys Val Gly
        35                  40                  45

<210>  25
<211>  49
<212>  PRT
<213>  Artificial Sequence

<220>
<223>  Cys-Myrcludex B-Cys

<400>  25

Cys Gly Thr Asn Leu Ser Val Pro Asn Pro Leu Gly Phe Phe Pro Asp
1               5                   10                  15

His Gln Leu Asp Pro Ala Phe Gly Ala Asn Ser Asn Asn Pro Asp Trp
            20                  25                  30

Asp Phe Asn Pro Asn Lys Asp His Trp Pro Glu Ala Asn Lys Val Gly
        35                  40                  45

Cys

<210>  26
<211>  50
<212>  PRT
<213>  Artificial Sequence

<220>
<223>  Cys- Myrcludex B-Cys-D-Tyr

<400>  26

Cys Gly Thr Asn Leu Ser Val Pro Asn Pro Leu Gly Phe Phe Pro Asp
1               5                   10                  15

His Gln Leu Asp Pro Ala Phe Gly Ala Asn Ser Asn Asn Pro Asp Trp
            20                  25                  30

Asp Phe Asn Pro Asn Lys Asp His Trp Pro Glu Ala Asn Lys Val Gly
        35                  40                  45

Cys Tyr
    50

<210>  27
<211>  8

<212>  PRT
<213>  Artificial Sequence

<220>
<223>  HBVpreS9-16 with Xaa = L

<400>  27

Asn Pro Leu Gly Phe Leu Pro Asp
1               5


<210>  28
<211>  9
<212>  PRT
<213>  Artificial Sequence

<220>
<223>  HBVpreS8-16 with Xaa = L

<400>  28

Pro Asn Pro Leu Gly Phe Leu Pro Asp
1               5


<210>  29
<211>  9
<212>  PRT
<213>  Artificial Sequence

<220>
<223>  HBVpreS9-17 with Xaa = L

<400>  29

Asn Pro Leu Gly Phe Leu Pro Asp His
1               5


<210>  30
<211>  10
<212>  PRT
<213>  Artificial Sequence

<220>
<223>  HBVpreS8-17 with Xaa = L

<400>  30

Pro Asn Pro Leu Gly Phe Leu Pro Asp His
1               5                   10


<210>  31
<211>  20
<212>  PRT
<213>  Artificial Sequence

<220>
<223>  HBVpreS2-21 with Xaa = L

<400>  31

Gly Thr Asn Leu Ser Val Pro Asn Pro Leu Gly Phe Leu Pro Asp His
1               5                   10                  15

Gln Leu Asp Pro
            20

<210>  32
<211>  47
<212>  PRT
<213>  Artificial Sequence

<220>
<223>  HBVpreS2-48 with Xaa = L

<400>  32

Gly Thr Asn Leu Ser Val Pro Asn Pro Leu Gly Phe Leu Pro Asp His
1               5                   10                  15

Gln Leu Asp Pro Ala Phe Gly Ala Asn Ser Asn Asn Pro Asp Trp Asp
            20                  25                  30

Phe Asn Pro Asn Lys Asp His Trp Pro Glu Ala Asn Gln Val Gly
        35                  40                  45

<210>  33
<211>  47
<212>  PRT
<213>  Artificial Sequence

<220>
<223>  amino acid residues of Myrcludex B with Xaa = L

<400>  33

Gly Thr Asn Leu Ser Val Pro Asn Pro Leu Gly Phe Leu Pro Asp His
1               5                   10                  15

Gln Leu Asp Pro Ala Phe Gly Ala Asn Ser Asn Asn Pro Asp Trp Asp
            20                  25                  30

Phe Asn Pro Asn Lys Asp His Trp Pro Glu Ala Asn Lys Val Gly
        35                  40                  45

<210>  34
<211>  8
<212>  PRT
<213>  Artificial Sequence

<220>
<223>  Amino acid sequence P' with first Xaa = F or L or a natural or
       artificial amino acid with comparable physicochemical side chain
       properties and second Xaa = an amino acid with an acidic side
       chain, preferably D or E

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<400>  34

Asn Pro Leu Gly Phe Xaa Pro Xaa
1               5


<210>  35
<211>  9
<212>  PRT
<213>  Artificial Sequence

<220>
<223>  linear amino acid sequence of cyclo-intra[NCLGFFCD]y, wherein Y =
       D-Tyr

<400>  35

Asn Cys Leu Gly Phe Phe Cys Asp Tyr
1               5


<210>  36
<211>  9
<212>  PRT
<213>  Artificial Sequence

<220>
<223>  amino acid sequence of cyclo[NPLGFFPDK] which is preS9-16-Lys

<400>  36

Asn Pro Leu Gly Phe Phe Pro Asp Lys
1               5


<210>  37
<211>  11
<212>  PRT
<213>  Artificial Sequence

<220>
<223>  amino acid sequence of cyclo[CNPLGFFPDCK], which is
       Cys-preS9-16-Cys-Lys

<400>  37

Cys Asn Pro Leu Gly Phe Phe Pro Asp Cys Lys
1               5                   10


<210>  38

<211>  10
<212>  PRT
<213>  Artificial Sequence

<220>
<223>  amino acid sequence of cyclo[KNPLGFFPDy], which is
       Lys-preS9-16-(D-Tyr)

<400>  38

Lys Asn Pro Leu Gly Phe Phe Pro Asp Tyr
1               5                   10


<210>  39
<211>  119
<212>  PRT
<213>  Hepatitis B virus

<400>  39

Met Gly Gly Trp Ser Ser Lys Pro Arg Lys Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Gly Ala Asn Ser Asn Asn Pro Asp Trp Asp Phe Asn Pro Ile
        35                  40                  45

Lys Asp His Trp Pro Gln Ala Asn Gln Val Gly Val Gly Ala Phe Gly
    50                  55                  60

Pro Gly Phe Thr Pro Pro His Gly Gly Val Leu Gly Trp Ser Pro Gln
65                  70                  75                  80

Ala Gln Gly Ile Leu Ala Thr Val Pro Ala Met Pro Pro Pro Ala Ser
                85                  90                  95

Thr Asn Arg Gln Ser Gly Arg Gln Pro Thr Pro Ile Ser Pro Pro Leu
            100                 105                 110

Arg Asp Ser His Pro Gln Ala
        115


<210>  40
<211>  119
<212>  PRT
<213>  Hepatitis B virus

<400>  40

Met Gly Gly Trp Ser Ser Lys Pro Arg Lys Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Lys Ala Asn Ser Glu Asn Pro Asp Trp Asp Leu Asn Pro His
        35                  40                  45

Lys Asp Asn Trp Pro Asp Ala His Lys Val Gly Val Gly Ala Phe Gly
    50                  55                  60

Pro Gly Phe Thr Pro Pro His Gly Gly Leu Leu Gly Trp Ser Pro Gln
65                  70                  75                  80

Ala Gln Gly Ile Leu Thr Ser Val Pro Ala Ala Pro Pro Pro Ala Ser
                85                  90                  95

Thr Asn Arg Gln Ser Gly Arg Gln Pro Thr Pro Leu Ser Pro Pro Leu
            100                 105                 110

Arg Asp Thr His Pro Gln Ala
        115

<210>  41
<211>  119
<212>  PRT
<213>  Hepatitis B virus

<400>  41

Met Gly Gly Trp Ser Ser Lys Pro Arg Lys Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Lys Ala Asn Ser Glu Asn Pro Asp Trp Asp Leu Asn Pro His
        35                  40                  45

Lys Asp Asn Trp Pro Asp Ala His Lys Val Gly Val Gly Ala Phe Gly
    50                  55                  60

Pro Gly Phe Thr Pro Pro His Gly Gly Leu Leu Gly Trp Ser Pro Gln
65                  70                  75                  80

Ala Gln Gly Ile Leu Thr Ser Val Pro Ala Ala Pro Pro Pro Ala Ser
                85                  90                  95

Thr Asn Arg Gln Ser Gly Arg Gln Pro Thr Pro Leu Ser Pro Pro Leu
            100                 105                 110

Arg Asp Thr His Pro Gln Ala
        115

<210>  42
<211>  108
<212>  PRT
<213>  Hepatitis B virus

<400>  42

Met Gly Gln Asn Leu Ser Thr Ser Asn Pro Leu Gly Phe Phe Pro Glu
1               5                   10                  15

His Gln Leu Asp Pro Ala Phe Lys Ala Asn Thr Asn Asn Pro Asp Trp
            20                  25                  30

Asp Phe Asn Pro Lys Lys Asp Tyr Trp Pro Glu Ala Asn Lys Val Gly
        35                  40                  45

Ala Gly Ala Phe Gly Pro Gly Phe Thr Pro Pro His Gly Gly Leu Leu
    50                  55                  60

Gly Trp Ser Pro Gln Ala Gln Gly Ile Leu Thr Thr Leu Pro Ala Asn
65                  70                  75                  80

Pro Pro Pro Ala Ser Thr Asn Arg Gln Ser Gly Arg Gln Pro Thr Pro
                85                  90                  95

Leu Ser Pro Pro Leu Arg Asp Thr His Pro Gln Ala
            100                 105

<210>  43
<211>  107
<212>  PRT
<213>  Hepatitis B virus

<400>  43

Met Gly Gln Asn Leu Ser Thr Ser Asn Pro Leu Gly Phe Phe Pro Asp
1               5                   10                  15

His Gln Leu Asp Pro Ala Phe Arg Ala Asn Thr Asn Asn Pro Asp Trp
            20                  25                  30

Asp Phe Asn Pro Asn Lys Asp Thr Trp Pro Asp Ala Asn Lys Val Gly
        35                  40                  45

Ala Gly Ala Phe Gly Leu Gly Phe Thr Pro Pro His Gly Gly Leu Leu
    50                  55                  60

Gly Trp Ser Pro Gln Ala Gln Gly Phe Gln Thr Leu Pro Ala Asn Pro
65                  70                  75                  80

Pro Pro Ala Ser Thr Asn Arg Gln Ser Gly Arg Gln Pro Thr Pro Leu
                85                  90                  95

Ser Pro Pro Leu Arg Thr Thr His Pro Gln Ala
            100                 105

<210>  44
<211>  118
<212>  PRT
<213>  Hepatitis B virus

<400>  44

Met Gly Leu Ser Trp Thr Val Pro Leu Glu Trp Gly Lys Asn Ile Ser
1               5                   10                  15

Thr Thr Asn Pro Leu Gly Phe Phe Pro Asp His Gln Leu Asp Pro Ala
            20                  25                  30

Phe Arg Ala Asn Thr Arg Asn Pro Asp Trp Asp His Asn Pro Asn Lys
        35                  40                  45

Asp His Trp Thr Glu Ala Asn Lys Val Gly Val Gly Ala Phe Gly Pro
    50                  55                  60

Gly Phe Thr Pro Pro His Gly Gly Leu Leu Gly Trp Ser Pro Gln Ala
65                  70                  75                  80

Gln Gly Met Leu Lys Thr Leu Pro Ala Asp Pro Pro Pro Ala Ser Thr
                85                  90                  95

Asn Arg Gln Ser Gly Arg Gln Pro Thr Pro Ile Thr Pro Pro Leu Arg
            100                 105                 110

Asp Thr His Pro Gln Ala
        115

<210>  45
<211>  119
<212>  PRT
<213>  Hepatitis B virus

<400>  45

Met Gly Ala Pro Leu Ser Thr Thr Arg Arg Gly Met Gly Gln Asn Leu
1               5                   10                  15

Ser Val Pro Asn Pro Leu Gly Phe Phe Pro Asp His Gln Leu Asp Pro

20                  25                  30

Leu Phe Arg Ala Asn Ser Ser Ser Pro Asp Trp Asp Phe Asn Thr Asn
        35                  40                  45

Lys Asp Ser Trp Pro Met Ala Asn Lys Val Gly Val Gly Gly Tyr Gly
    50                  55                  60

Pro Gly Phe Thr Pro Pro His Gly Gly Leu Leu Gly Trp Ser Pro Gln
65                  70                  75                  80

Ala Gln Gly Val Leu Thr Thr Leu Pro Ala Asp Pro Pro Pro Ala Ser
                85                  90                  95

Thr Asn Arg Arg Ser Gly Arg Lys Pro Thr Pro Val Ser Pro Pro Leu
            100                 105                 110

Arg Asp Thr His Pro Gln Ala
        115

<210>  46
<211>  118
<212>  PRT
<213>  Hepatitis B virus

<400>  46

Met Gly Leu Ser Trp Thr Val Pro Leu Glu Trp Gly Lys Asn Leu Ser
1               5                   10                  15

Ala Ser Asn Pro Leu Gly Phe Leu Pro Asp His Gln Leu Asp Pro Ala
            20                  25                  30

Phe Arg Ala Asn Thr Asn Asn Pro Asp Trp Asp Phe Asn Pro Lys Lys
        35                  40                  45

Asp Pro Trp Pro Glu Ala Asn Lys Val Gly Val Gly Ala Tyr Gly Pro
    50                  55                  60

Gly Phe Thr Pro Pro His Gly Gly Leu Leu Gly Trp Ser Pro Gln Ser
65                  70                  75                  80

Gln Gly Thr Leu Thr Thr Leu Pro Ala Asp Pro Pro Pro Ala Ser Thr
                85                  90                  95

Asn Arg Gln Ser Gly Arg Gln Pro Thr Pro Ile Ser Pro Pro Leu Arg
            100                 105                 110

Asp Ser His Pro Gln Ala

        115

<210>  47
<211>  108
<212>  PRT
<213>  Hepatitis B virus

<400>  47

Met Gly Gln Asn His Ser Val Thr Asn Pro Leu Gly Phe Phe Pro Asp
1               5                   10                  15

His Gln Leu Asp Pro Leu Phe Arg Ala Asn Ser Asn Asn Pro Asp Trp
            20                  25                  30

Asp Phe Asn Pro Asn Lys Asp Thr Trp Pro Glu Ala Thr Lys Val Gly
        35                  40                  45

Val Gly Ala Phe Gly Pro Gly Phe Thr Pro Pro His Gly Gly Leu Leu
    50                  55                  60

Gly Trp Ser Pro Gln Ala Gln Gly Ile Leu Thr Thr Leu Pro Ala Ala
65                  70                  75                  80

Pro Pro Pro Ala Ser Thr Asn Arg Gln Ser Gly Arg Lys Ala Thr Pro
                85                  90                  95

Ile Ser Pro Pro Leu Arg Asp Thr His Pro Gln Ala
            100                 105


<210>  48
<211>  119
<212>  PRT
<213>  Hepatitis B virus

<400>  48

Met Gly Ala Pro Leu Ser Thr Ala Arg Arg Gly Met Gly Gln Asn Leu
1               5                   10                  15

Ser Val Pro Asn Pro Leu Gly Phe Phe Pro Asp His Gln Leu Asp Pro
            20                  25                  30

Leu Phe Arg Ala Asn Ser Ser Ser Pro Asp Trp Asp Phe Asn Thr Asn
        35                  40                  45

Lys Asp Asn Trp Pro Met Ala Asn Lys Val Gly Val Gly Gly Phe Gly
    50                  55                  60

Pro Gly Phe Thr Pro Pro His Gly Gly Leu Leu Gly Trp Ser Pro Gln
65                  70                  75                  80

Ala Gln Gly Ile Leu Thr Thr Ser Pro Pro Asp Pro Pro Pro Ala Ser
                85                  90                  95

Thr Asn Arg Arg Ser Gly Arg Lys Pro Thr Pro Val Ser Pro Pro Leu
            100                 105                 110

Arg Asp Thr His Pro Gln Ala
        115

<210>  49
<211>  108
<212>  PRT
<213>  Hepatitis B virus

<400>  49

Met Gly Gln Asn Leu Ser Val Ser Asn Pro Leu Gly Phe Phe Pro Glu
1               5                   10                  15

His Gln Leu Asp Pro Leu Phe Arg Ala Asn Thr Asn Asn Pro Asp Trp
            20                  25                  30

Asp Phe Asn Pro Asn Lys Asp Thr Trp Pro Glu Ala Thr Lys Val Gly
        35                  40                  45

Val Gly Ala Phe Gly Pro Gly Phe Thr Pro Pro His Gly Gly Leu Leu
    50                  55                  60

Gly Trp Ser Pro Gln Ala Gln Gly Val Thr Thr Ile Leu Pro Ala Val
65                  70                  75                  80

Pro Pro Pro Ala Ser Thr Asn Arg Gln Ser Gly Arg Gln Pro Thr Pro
                85                  90                  95

Ile Ser Pro Pro Leu Arg Asp Thr His Pro Gln Ala
            100                 105

<210>  50
<211>  105
<212>  PRT
<213>  Hepatitis B virus

<400>  50

Met Gly Leu Asn Gln Ser Thr Phe Pro Leu Gly Phe Phe Pro Ser His
1               5                   10                  15

Gln Leu Asp Pro Leu Phe Lys Ala Asn Ala Gly Ser Ala Asp Trp Asp
            20                  25                  30

Lys Pro Lys Asp Pro Trp Pro Gln Ala His Asp Thr Ala Val Gly Ala
        35                  40                  45

Phe Gly Pro Gly Leu Val Pro Pro His Gly Gly Leu Leu Gly Trp Ser
    50                  55                  60

Ser Gln Ala Gln Gly Leu Ser Val Thr Val Pro Asp Thr Pro Pro Pro
65                  70                  75                  80

Pro Ser Thr Asn Arg Asp Lys Gly Arg Lys Pro Thr Pro Ala Thr Pro
                85                  90                  95

Pro Leu Arg Asp Thr His Pro Gln Ala
            100                 105

<210>  51
<211>  59
<212>  PRT
<213>  Artificial Sequence

<220>
<223>  HBV preS consensus sequence (for amino acid positions (-11) to
       48)

<400>  51

Met Gly Gly Trp Ser Ser Thr Pro Arg Lys Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Arg Ala Asn Ser Asn Asn Pro Asp Trp Asp Phe Asn Pro Asn
        35                  40                  45

Lys Asp His Trp Pro Glu Ala Asn Lys Val Gly
    50                  55

## Description

The present invention relates to cyclic preS-derived peptides of HBV which are versatile vehicles for the targeted delivery of compounds to the liver, preferably to hepatocytes, *in vitro* as well as *in vivo.* The present invention further relates to conformationally constrained cyclic preS-derived peptides of HBV which are versatile vehicles for the targeted delivery of compounds to the liver, preferably to hepatocytes, *in vitro* as well as *in vivo.*

The present invention further relates to pharmaceutical compositions comprising at least one cyclic peptide. The present invention further relates to medical uses of said cyclic peptides and the pharmaceutical compositions, such as in the diagnosis, prevention and/or treatment of a liver disease or condition. The present invention further relates to methods for the specific delivery of compound(s) to the liver, methods for the diagnosis, prevention and/or treatment of a liver disease or condition and/or methods for the diagnosis, prevention and/or treatment of a cardiovascular disease.

### BACKGROUND OF THE INVENTION

The liver, an organ which is present in vertebrates and other animals, plays a major role in the metabolism and has a number of functions in the body, including glycogen storage, decomposition of red blood cells, synthesis of plasma proteins, and detoxification. The liver also is the largest gland in the human body. It lies below the diaphragm in the thoracic region of the abdomen. It produces bile, an alkaline compound which aids in digestion, via the emulsification of lipids. It also performs and regulates a wide variety of high-volume biochemical reactions requiring specialized tissues.

Hepatocytes make up 70 to 80% of the cytoplasmic mass of the liver. Hepatocytes are involved in protein synthesis, protein storage and transformation of carbohydrates, synthesis of cholesterol, bile salts and phospholipids, and detoxification, modification and excretion of exogenous and endogenous substances. The hepatocyte also initiates the formation and secretion of bile.

There is a wide number of known liver diseases, such as:
- Hepatitis: inflammation of the liver, caused mainly by various viruses but also by certain poisons, autoimmunity or hereditary conditions;
- Cirrhosis: the formation of fibrous tissue in the liver, replacing dead liver cells. The death of the liver cells can for example be caused by viral hepatitis, alcoholism or contact with other liver-toxic chemicals;
- Haemochromatosis: a hereditary disease causing the accumulation of iron in the body, eventually leading to liver damage;
- Cancer of the liver: primary hepatocellular carcinoma (HCC) or cholangiocarcinoma and metastatic cancers, usually from other parts of the gastrointestinal tract;
- Wilson's disease: a hereditary disease which causes the body to retain copper;
- Primary sclerosing cholangitis: an inflammatory disease of the bile duct, autoimmune in nature;
- Primary biliary cirrhosis: autoimmune disease of small bile ducts;
- Budd-Chiari syndrome: obstruction of the hepatic vein;
- Gilbert's syndrome: a genetic disorder of bilirubin metabolism, found in about 5% of the population;
- Glycogen storage disease type II: the build-up of glycogen causes progressive muscle weakness (myopathy) throughout the body and affects various body tissues, particularly in the heart, skeletal muscles, liver and nervous system.

There are also many pediatric liver diseases, such as biliary atresia, alpha-1-antitrypsin deficiency, alagille syndrome, and progressive familial intrahepatic cholestasis; as well as metabolic diseases.

Furthermore, several pathogens and parasites, especially of tropical diseases, have a liver stage during their life cycle.

For instance malaria, which is one of the most common infectious diseases and an enormous public-health problem. Malaria is caused by protozoan parasites of the genus *Plasmodium.* The most serious forms of the disease are caused by *Plasmodium falciparum* and *Plasmodium vivax,* but other related species (*Plasmodium ovale, Plasmodium malariae,* and sometimes *Plasmodium knowlesi*) can also infect humans. This group of human-pathogenic *Plasmodium* species is usually referred to as malaria parasites. Malaria parasites are transmitted by female *Anopheles* mosquitoes. Malaria in humans develops via two phases: an exoerythrocytic (hepatic phase or "liver stage") and an erythrocytic phase. When an infected mosquito pierces a person's skin to take a blood meal, sporozoites in the mosquito's saliva enter the bloodstream and migrate to the liver. Within about 30 minutes of being introduced into the human host, they infect hepatocytes, multiplying asexually and asymptomatically for a period of 6-15 days. Once in the liver these organisms differentiate to yield thousands of merozoites which, following rupture of their host cells, escape into the blood and infect red blood cells, thus beginning the erythrocytic stage of the life cycle. The parasite escapes from the liver undetected by wrapping itself in the cell membrane of the infected host liver cell. The parasite is relatively protected from attack by the body's immune system because for most of its human life cycle it resides within the liver and blood cells and is relatively invisible to immune surveillance. There is increasing interest in developing drugs that specifically address the liver specific stages of malaria parasites (e.g. primaquin) in order to obviate the development of blood stages.

Another example is schistosomiasis or bilharziosis, which is a parasitic disease caused by several species of flatworm. Although it has a low mortality rate, schistosomiasis can be very debilitating. It is an often chronic illness that results from infection of the blood with a parasitic flatworm (schistosome). It causes debilitation and causes liver and intestinal damage. It is most commonly found in Asia, Africa, and South America, especially in areas with water that is contaminated with fresh water snails, which contain the parasite.

Hepatitis B virus (HBV), the cause for hepatitis B, is the prototype of a family of small, enveloped DNA viruses of mammals and birds (Seeger and Mason, 2000). The HBV envelope encloses three proteins termed L-(large), M-(middle) and S-(small). They share the C-terminal S-domain with four transmembrane regions. The M- and L-protein carry additional N-terminal extensions of 55 and, genotype-dependent, 107 or 118 aa (preS2- and preS1). In virions the stoichiometric ratio of L, M and S is about 1:1:4, while the more abundantly secreted non-infectious subviral particles (SVPs) contain almost exclusively Sand only traces of L-protein (Nassal, 1996). During synthesis, the preS1-domain of L is myristoylated and at some point of the HBV life cycle translocated through the ER-derived membrane. This modification is essential for infectivity (Gripon et al., 1995; Le Seyec et al., 1999). HBV as well as the other hepatotropic viruses have a liver tropism, i.e. the liver is the tissue which specifically supports the growth of HBV.

US 7,001,760 B2 disclose recombinant vectors derived from hepatitis B virus (HBV), which can be used for gene therapy, such as the delivery of therapeutic genes to liver cells and the expression of heterologous genes in liver cells. However, HBV vectors have a limited capacity for packaging genes.

In WO 2009/092612 and WO 2012/107579, whose contents are incorporated herewith by reference in its entirety, the inventors describe hydrophobic modified preS-derived peptides of HBV and their use as vehicles for the specific delivery of compounds to the liver.

There is a need for universal diagnostic, therapeutic and/or preventative approaches that in a subject specifically target the organ liver and specifically deliver a desired compound or drug to the liver

The present invention, thus, aims to provide improved means and methods for the effective, specific targeted diagnosis, prevention and/or treatment of liver diseases and the regulation of the liver function.

### SUMMARY OF THE INVENTION

According to the present invention this object is solved by providing a cyclic peptide of the general formula I

**(X)ₘ**-**P**-**(Y)ₙ** **(I)**

wherein
**P** is the amino acid sequence NPLGFXaaP (SEQ. ID NO: 1), with Xaa being F or L,
**X** is an amino acid sequence having a length of **m** amino acids, s
   wherein **m** is 0 or at least 1;
**Y** is an amino sequence having a length of **n** amino acids,
   wherein **n** is 0 or at least 1;
   and wherein **m** + **n** is 0 or at least 1;
or a pharmaceutically acceptable salt thereof,
for use in liver targeting.

According to the present invention this object is solved by using the cyclic peptides of the present invention having the general formula I as vehicle or shuttle for the specific delivery of compound(s) to the liver.

According to the present invention this object is solved by providing a pharmaceutical composition comprising
(i) at least one cyclic peptide of the present invention (of the general formula I),
(ii) optionally, a pharmaceutically acceptable carrier and/or excipient.

According to the present invention this object is solved by providing the cyclic peptide of the present invention or the pharmaceutical composition of the present invention (of the general formula I) for use in the diagnosis, prevention and/or treatment of a liver disease or condition.

According to the present invention this object is solved by providing the cyclic peptide of the present invention (of the general formula I) or the pharmaceutical composition of the present invention for use in the diagnosis, prevention and/or treatment of a cardiovascular disease (CVD).

According to the present invention this object is solved by a method for the specific delivery of compound(s) to the liver, comprising the administration of a therapeutically effective amount of a cyclic peptide of the present invention (of the general formula I) or a pharmaceutical composition of the present invention.

According to the present invention this object is solved by a method for the diagnosis, prevention and/or treatment of a liver disease or condition, comprising the administration of a therapeutically effective amount of a cyclic peptide of the present invention (of the general formula I) or a pharmaceutical composition of the present invention.

According to the present invention this object is solved by a method for the diagnosis, prevention and/or treatment of a cardiovascular disease (CVD), comprising the administration of a therapeutically effective amount of a cyclic peptide of the present invention (of the general formula I) or a pharmaceutical composition of the present invention.

According to the present invention this object is solved by providing a cyclic peptide having the general formula II

**Rₚ**-**(X)ₘ**-**P'-(Y)ₙ-(Z)₀**-**R_{q}** **(II)**

wherein
**P'** is the amino acid sequence NPLGFxₐx_{b}P (SEQ. ID NO: 34),
   **xₐ** is F or L or a natural or artificial amino acid with comparable
physicochemical side chain properties,
   **x_{b}** is an amino acid with an acidic side chain, preferably D or E,
**X** is an amino acid sequence having a length of **m** amino acids,
   wherein **m** is 0 or 1;
**Y** is an amino sequence having a length of **n** amino acids,
   wherein **n** is 0, 1, 2 or at most 3;
   and wherein **m** + **n** ≥ 0;
**Z** is an amino acid sequence of **o** amino acids,
   wherein **o** is 0 or 1,
   and which comprises a reactive side chain, e.g. amine, thiol, acidic side chain, hydroxyl, azid,
      such as aminoproline,
**Rₚ** is a hydrophobic moiety
   **p** is 0 or at least 1,
**R_{q}** is a hydrophobic moiety
   **q** is 0 or at least 1,
   and wherein **p** + **q** ≥ 1;
or a pharmaceutically acceptable salt thereof.

According to the present invention this object is solved by providing a pharmaceutical composition comprising
(i) at least one cyclic peptide of the present invention (of the general formula II),
(ii) optionally, a pharmaceutically acceptable carrier and/or excipient.

According to the present invention this object is solved by providing the cyclic peptide of the present invention (of the general formula II) or the pharmaceutical composition of the present invention for use in medicine.

According to the present invention this object is solved by providing a cyclic peptide having the general formula II for use in liver targeting, such as for the specific delivery of compound(s) to the liver.

According to the present invention this object is solved by using the cyclic peptides of the present invention having the general formula II as vehicle or shuttle for the specific delivery of compound(s) to the liver.

According to the present invention this object is solved by providing the cyclic peptide of the present invention (of the general formula II) or the pharmaceutical composition of the present invention for use in the diagnosis, prevention and/or treatment of a liver disease or condition.

According to the present invention this object is solved by providing the cyclic peptide of the present invention (of the general formula II) or the pharmaceutical composition of the present invention for use in the diagnosis, prevention and/or treatment of a cardiovascular disease (CVD).

According to the present invention this object is solved by a method for the specific delivery of compound(s) to the liver, comprising the administration of a therapeutically effective amount of a cyclic peptide of the present invention (of the general formula II) or a pharmaceutical composition of the present invention.

According to the present invention this object is solved by a method for the diagnosis, prevention and/or treatment of a liver disease or condition, comprising the administration of a therapeutically effective amount of a cyclic peptide of the present invention or a pharmaceutical composition of the present invention.

According to the present invention this object is solved by a method for the diagnosis, prevention and/or treatment of a cardiovascular disease (CVD), comprising the administration of a therapeutically effective amount of a cyclic peptide of the present invention (of the general formula II) or a pharmaceutical composition of the present invention.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "7 to 49" should be interpreted to include not only the explicitly recited values of 4 to 19, but also include individual values and sub-ranges within the indicated range. Thus, included in this numerical range are individual values such as 7, 8, 9, 10, 11, 12, 13, 14, 15, 16 ... 37, 38, 39, 40, 41, 42, 43, 44, 45, 46, 47, 48, 49, and sub-ranges such as from 7 to 10, from 10 to 15, from 15 to 25, from 28 to 39, from 35 to 47, from 35 to 49 etc. This same principle applies to ranges reciting only one numerical value, such as "at least 1 or "at least one amino acid". Furthermore, such an interpretation should apply regardless of the breadth of the range or the characteristics being described.

### Cyclic HBV preS-derived peptides for use in liver targeting

As outlined above, the present invention provides cyclic peptides for use in liver targeting.

The cyclic peptides of the present invention are subject of the parallel European patent application No. 15200494.1 filed December 16,2015.

Said cyclic peptides are preS-derived peptides of hepatitis B virus (HBV).

A cyclic peptide of the present invention has the general formula I

**(X)ₘ**-**P-(Y)ₙ** **(I)**

wherein
**P** is the amino acid sequence NPLGFXaaP (SEQ. ID NO: 1), with Xaa being F or L,
**X** is an amino acid sequence having a length of **m** amino acids,
   wherein **m** is 0 or at least 1;
**Y** is an amino sequence having a length of **n** amino acids,
   wherein **n** is 0 or at least 1;
   and wherein **m** + **n** is 0 or at least 1;
or a pharmaceutically acceptable salt thereof.

Preferably, m + n is 0 to 42,
and/or wherein m = 0 to 8 and/or n = 0 to 34, provided that m + n is 0 or at least 1.

In an embodiment with
m + n = 0, the peptide has a length of / contains 7 amino acids (namely P);
m + n = 1, the peptide has a length of / contains 8 amino acids;
m + n = 2, the peptide has a length of / contains 9 amino acids;
m + n = 3, the peptide has a length of / contains 10 amino acids;
m + n = 4, the peptide has a length of / contains 11 amino acids;
m + n = 5, the peptide has a length of / contains 12 amino acids;
m + n = 6, the peptide has a length of / contains 13 amino acids;
m + n = 7, the peptide has a length of / contains 14 amino acids;
m + n = 8, the peptide has a length of / contains 15 amino acids;
m + n = 15, the peptide has a length of / contains 22 amino acids;
m + n = 40, the peptide has a length of / contains 47 amino acids;
m + n = 42, the peptide has a length of / contains 49 amino acids.

### - Cyclization

The cyclic peptides of the present invention can be cyclized in different ways, preferably
(a) via thiol oxidation (disulfide bridge formation) of two cysteines (C) comprised in the peptide,
   such as via a C at or near the N-terminus and a C at or near the C-terminus (of the peptide sequence before cyclization);
(b) amide condensation of two amino acid side chains (lactam),
   such as via a lysine (K) side chain and an aspartic acid (D) side chain,
   such as
   via a K side chain at or near the N-terminus and a D side chain at or near the C-terminus (of the peptide sequence before cyclization);
(c) via head-to-tail cyclization,
   such as
   via a lysine (K) at the N-terminus and the C-terminus (of the peptide sequence before cyclization),
   via the N-terminus, i.e. the amino group of the N-terminal amino acid and the C-terminus, i.e. carboxyl group of the C-terminal amino acid (of the peptide sequence before cyclization),
(d) via backbone cyclization,
(e) via thioether formation,
   and/or
(f) via hydrogen bond formation and/or bond-forming derivatives of amino acids,
   such as amino acids forming a tryptophan zipper.

In one embodiment, the cyclic peptides of the present invention do not comprise peptides which are cyclized within the amino acid sequence of P (i.e. within the pharmacophore of 7 amino acids, of SEQ ID NO. 1 (NPLGFXaaP with Xaa = F or L)). i.e. via amino acid side chains of P.

However, the P sequence can be cyclized via head-to-tail cyclization.

For example, in one embodiment, the cyclic peptide comprises P NPLGFXaaP with Xaa = F, i.e. NPLGFFP (SEQ. ID NO: 2), and m and n are each 0. The peptide is cyclized via head-to-tail cyclization via the N-terminus, i.e. the amino group of the N-terminal asparagine (N) and the C-terminus, i.e. carboxyl group of the C-terminal amino acid proline (P).

In one embodiment the cyclic peptide comprises P NPLGFXaaP with Xaa = F, i.e. NPLGFFP (SEQ. ID NO: 2), and X = Cys and Y = Cys, m and n are each 1. The peptide can be cyclized via thiol oxidation (disulfide bridge formation) of the two cysteines (C).

In one embodiment the cyclic peptide comprises P NPLGFXaaP with Xaa = F, i.e. NPLGFFP (SEQ. ID NO: 2), and X = Cys and Y = Asp-Cys, m = 1 and n = 2. The peptide can be cyclized via thiol oxidation (disulfide bridge formation) of the two cysteines (C).

In one embodiment the cyclic peptide comprises P NPLGFXaaP with Xaa = F, i.e. NPLGFFP (SEQ. ID NO: 2), and X = Cys and Y = His-Asp-Cys, m = 1 and n = 3. The peptide can be cyclized via thiol oxidation (disulfide bridge formation) of the two cysteines (C).

In one embodiment the cyclic peptide comprises P NPLGFXaaP with Xaa = F, i.e. NPLGFFP (SEQ. ID NO: 2), and X = Cys-Pro and Y = Asp-Cys, m = 2 and n = 2. The peptide can be cyclized via thiol oxidation (disulfide bridge formation) of the two cysteines (C).

In one embodiment the cyclic peptide comprises P NPLGFXaaP with Xaa = F, i.e. NPLGFFP (SEQ. ID NO: 2), and X = Cys-Pro and Y = His-Asp-Cys, m = 2 and n = 3. The peptide can be cyclized via thiol oxidation (disulfide bridge formation) of the two cysteines (C).

In one embodiment the cyclic peptide comprises the Myrcludex B sequence with P NPLGFXaaP with Xaa = F, i.e. NPLGFFP (SEQ. ID NO: 2), and m = 7 and n = 33. The peptide can be cyclized via head-to-tail cyclization via the N-terminus, i.e. the amino group of the N-terminal glycine (G) and the C-terminus, i.e. carboxyl group of the C-terminal amino acid glycine (G).

In one embodiment the cyclic peptide comprises the Myrcludex B sequence with P NPLGFXaaP with Xaa = F, i.e. NPLGFFP (SEQ. ID NO: 2), and two additional cysteines, such that m = 8 and n = 34. The peptide can be cyclized via thiol oxidation (disulfide bridge formation) of the two cysteines (C).

In other embodiments, the cyclic peptides comprise P NPLGFXaaP with Xaa = L, i.e. NPLGFLP (SEQ. ID NO: 3), respectively, as well as optionally the further additional amino acids, as described above.

In one embodiment, the cyclic peptides of the present invention comprise peptides which are cyclized within the amino acid sequence of P (i.e. within the pharmacophore of 7 amino acids, of SEQ ID NO. 1 (NPLGFXaaP with Xaa = F or L)). i.e. via amino acid side chains of P.

### - Hydrophobic modification

In a preferred embodiment, the cyclic peptide of the present invention is hydrophobically modified, i.e. it carries at least one covalently attached *hydrophobic modification.*

The position of the hydrophobic modification(s) can vary within the peptide sequence. Preferably, the hydrophobic modification(s) is/are at amino acid side chain(s) of X and/or Y.

In one embodiment, the hydrophobic modification(s) is/are at amino acid side chain(s) of P.

Preferably, the hydrophobic modification is an acylation and/or addition of hydrophobic moieties,
more preferably an acylation with C8 to C22 fatty acids ,
such as myristoyl (C 14), palmitoyl (C16) or stearoyl (C18),
even more preferably myristoyl (C 14),
or the hydrophobic moiety or moieties is selected from cholesterol, cholesterol derivatives, phospholipids, glycolipids, glycerol esters, steroids, ceramids, isoprene derivatives.

The cyclic peptides of the invention can carry more than one hydrophobic modification, such as two, three, four or more, which can be the same or different.
For example, a cyclic peptide of the invention carries one myristoyl group (C 14) and one stearoyl (C18) group.

Preferably, the cyclic peptides of the invention carry one or two hydrophobic modification(s).

In one embodiment, a cyclic peptide of the present invention has the general formula Ia

**cyclo[(X)ₘ**-**P-(Y)ₙ]** **(Ia)**

and carries at least one hydrophobic modification at amino acid side chain(s) of X and/or P.

General formula Ia can also be shown as

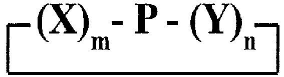

The cyclic peptide with general formula Ia can be obtained via head-to-tail cyclization, such as
- via thiol oxidation (disulfide bridge formation) of two cysteines (C) comprised in the peptide ,
   namely a cysteine at the N-terminus and a cysteine at or near the C-terminus (of the peptide sequence before cyclization)
- via a lysine (K) at the N-terminus or the N-terminus itself and the C-terminus (of the peptide sequence before cyclization).

For example, a cyclic peptide with general formula Ia can be:

**H**-**cyclo[(X)ₘ-P-(Y)ₙ]**

such as 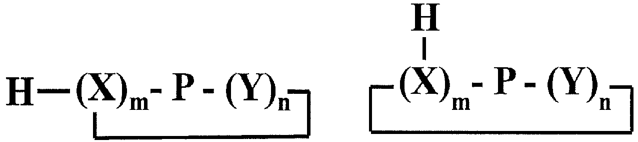 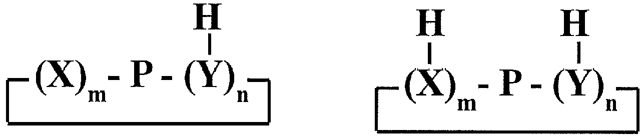 wherein **H** is the hydrophobic modification, preferably myristoyl (C 14).

For example

**myr-cyclo[(X)ₘ-P-(Y)ₙ]**

### - Liver targeting

The cyclic peptides of the present invention are used for liver targeting, such as the specific delivery of compound(s) to the liver.

The cyclic peptides of the present invention are suitable for liver targeting, because they either target NTCP (gene name SLC10A1), the sodium taurocholate cotransporter polypeptide (in particular the cyclic peptides that are not cyclized within P) or use an unknown mechanism to enrich in the liver.

The human sodium taurocholate cotransporter polypeptide NTCP/SLC10A1 is a HBV preS1-specific receptor which plays a key role in Hepatitis B virus (HBV) and/or Hepatitis D virus (HDV) infection, as it was recently identified by the inventors (see e.g. WO 2014/072526, WO 2014/072524 and WO 2015/014830). See also WO 2013/159243 A1. Expression of this receptor or of certain non-human counterparts allows to transform cells that were previously unable to specifically bind HBV and/or HDV and/or non-susceptible to HBV and/or HDV infection into cells that are HBV and/or HDV binding-competent and/or susceptible to HBV and/or HDV infection. Cells that became susceptible to HBV and/or HDV infection after differentiation (HepaRG cells) show a significantly increased susceptibility upon expression ofNTCP.

NTCP is an integral transmembrane protein, not expressed in HepG2, HuH7, induced in HepaRG cells after DMSO treatment (Kotani *et al.,* 2012) and down-modulated in primary hepatocytes during de-differentiation (Doring *et al.,* 2012).

As used herein, "targeting NTCP" or "NTCP targeting" refers to the specific binding of the cyclic peptides of the present invention to the NTCP receptor on respective liver cells/hepatocytes, preferably within a host (subject or patient) but also in some animals that express a binding competent NTCP although they do not support infection (e.g. mouse, rat, dog). Several ligands (e.g. Cyclosporin A) and substrates (e.g. bile salts, ezetimibe) have been characterized so far, which might serve a similar function like the peptides described here. However, these substances are known to also address other bile salt transporters from the SLC family and are therefore not considered specific (Urban et al., 2014).

Preferably, the cyclic peptide of the present invention comprises or consists of an amino acid sequence selected from the group of
(wherein P, SEQ ID NO. 1 is underlined)
- **amino acid residues 9 to 15** (SEQ. ID NO: 2) HBVpreS9-15 NPLGFFP
   and e.g. additional amino acids,
   such as comprising a N-terminal and a C-terminal cysteine (in the peptide sequence before cyclization)
   X = Cys, Y = Cys, m = 1 and n =1 1
      Cys-preS9-15-Cys C-NPLGFFP-C (SEQ. ID NO: 4)
   such as a C-terminal D-Tyr
      Cys-HBVpreS9-15-Cys-D-Tyr C-NPLGFFP-C-y (SEQ. ID NO: 5)
- **amino acid residues 9 to 16**
   HBVpreS9-16 NPLGFFPD (SEQ. ID NO: 6)
   and e.g. additional amino acids,
   such as comprising a N-terminal and a C-terminal cysteine (in the peptide sequence before cyclization)
   X = Cys, Y = Asp-Cys, m = 1 and n =2
   Cys-HBVpreS9-16-Cys C-NPLGFFPD-C (SEQ. ID NO: 7)
      such as a C-terminal D-Tyr
   Cys-HBVpreS9-16-Cys C-NPLGFFPD-C-y (SEQ. ID NO: 8)
- **amino acid residues 8 to 16**
   HBVpreS8-16 PNPLGFFPD (SEQ. ID NO: 9)
      and e.g. additional amino acids,
      such as comprising a N-terminal and a C-terminal cysteine (in the peptide sequence before cyclization)
      X = Cys-Pro, Y = Asp-Cys, m = 2 and n =2
   Cys-HBVpreS8-16-Cys C-PNPLGFFPD-C (SEQ. ID NO: 10)
      such as a C-terminal D-Tyr
   Cys-HBVpreS8-16-Cys-D-Tyr C-PNPLGFFPD-C-y (SEQ. ID NO: 11)
- **amino acid residues 9 to 17**
   HBVpreS9-17 NPLGFFPDH (SEQ. ID NO: 12)
      and e.g. additional amino acids,
      such as comprising a N-terminal and a C-terminal cysteine (in the peptide sequence before cyclization)
      X = Cys, Y = Asp-His-Cys, m = 1 and n =3
   Cys-HBVpreS9-17-Cys C-NPLGFFPDH-C (SEQ. ID NO: 13)
      such as a C-terminal D-Tyr
   Cys-HBVpreS9-17-Cys-D-Tyr C-NPLGFFPDH-C-y (SEQ. ID NO: 14)
- **amino acid residues 8 to 17**
   HBVpreS8-17 PNPLGFFPDH (SEQ.ID NO:15)
      and e.g. additional amino acids,
      such as comprising a N-terminal and a C-terminal cysteine (in the peptide sequence before cyclization)
      X = Cys-Pro, Y = Asp-His-Cys, m = 2 and n =3
         Cys-HBVpreS8-17-Cys C-PNPLGFFPDH-C (SEQ. ID NO: 16)
      such as a C-terminal D-Tyr
         Cys-HBVpreS8-17-Cys-D-Tyr C-PNPLGFFPDH-C-y (SEQ. ID NO: 17)
- **amino acid residues 2 to 21**
   HBVpreS2-21 GTNLSVPNPLGFFPDHQL*DP* (SEQ. ID NO: 18)
   and e.g. additional amino acids,
   such as comprising a N-terminal and a C-terminal cysteine (in the peptide sequence before cyclization)
   X = CGTNLSVP, Y = DHQLDPC, m = 8 and n =7
      Cys-HBVpreS2-21-CyS C-GTNLSVPNPLGFFPDHQL*DP*-C (SEQ. ID NO: 19)
   such as a C-terminal D-Tyr
      Cys-HBVpreS2-21-Cys-D-Tyr C-GTNLSVPNPLGFFPDHQL*DP*-C-y (SEQ. ID NO: 2O)
- **amino acid residues 2 to 48 of genotype C**
   HBVpreS2-48 GTNLSVPNPLGFFPDHQL*DP*A*FGANSNNPDWDFNPNKDHWPEANQVG* (SEQ. ID NO: 21)
   and e.g. additional amino acids,
   such as comprising a N-terminal and a C-terminal cysteine (in the peptide sequence before cyclization)
   X = CGTNLSVP, Y = DHQLDPAFGANSNNPDWDFNPNKDHWPEANQVGC,
   m = 8 and n =34
      Cys-HBVpreS2-48-Cys C-GTNLSVPNPLGFFPDHQL*DPAFGANSNNPDWDFNPNKDHWPEANQVG-C* (SEQ. ID NO: 22)
   such as a C-terminal D-Tyr
      Cys-HBVpreS2-48-Cys-D-Tyr
      C-GTNLSVPNPLGFFPDHQL*DPAFGANSNNPDWDFNPNKDHWPEANQVG-*C*-*y (SEQ. ID NO: 23)
- **amino acid residues of Myrcludex B**
   GTNLSVPNPLGFFPDHQL*DPAFGANSNNPDWDFNPNKDHWPEANKVG* (SEQ. ID NO: 24)
   and e.g. additional amino acids,
   such as comprising a N-terminal and a C-terminal cysteine (in the peptide sequence before cyclization)
   X = CGTNLSVP, Y = DHQLDPAFGANSNNPDWDFNPNKDHWPEANKVGC,
   m = 8 and n =34
      Cys-Myrcludex B-Cys C-GTNLSVPNPLGFFPDHQL*DPAFGANSNNPDWDFNPNKDHWPEANKVG-*C (SEQ. ID NO: 25)
   such as a C-terminal D-Tyr
      Cys- Myrcludex B-Cys-D-Tyr
      C-GTNLSVPNPLGFFP*DHQLDPAFGANSNNPDWDFNPNKDHWPEANKVG-*C-y (SEQ. ID NO: 26)

Examples of preferred cyclic peptides of the present invention are:
cyclo [NPLGFFP] (SEQ. ID NO: 2)
cyclo [NPLGFFPD] (SEQ. ID NO: 6)
cyclo [PNPLGFFPD] (SEQ. ID NO: 9)
cyclo [NPLGFFPDH] (SEQ. ID NO: 12)
cyclo[PNPLGFFPDH] (SEQ. ID NO: 15)
cyclo [GTNLSVPNPLGFFPDHQLDP] (SEQ. ID NO: 18)
cyclo [GTNLSVPNPLGFFPDHQLDPAFGANSNNPDWDFNPNKDHWPEANQVG] (SEQ. ID NO:21)
cyclo [GTNLSVPNPLGFFPDHQLDPAFGANSNNPDWDFNPNKDHWPEANKVG] (SEQ. ID NO:24)
or

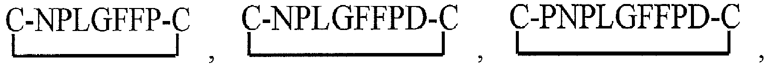

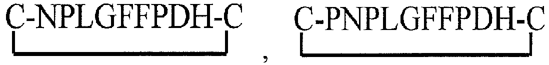

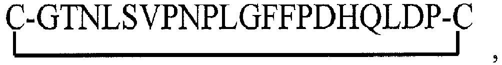

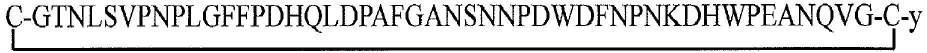

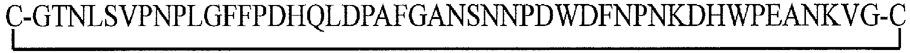

Preferably the above comprise a C-terminal D-amino acid, such as D-tyrosine, e.g.

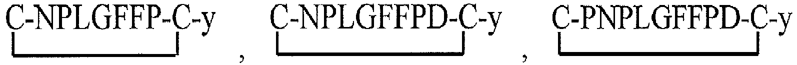

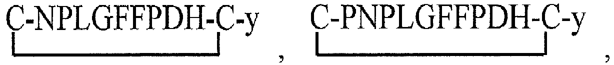

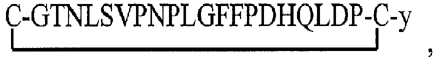

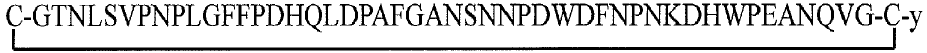

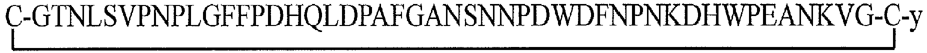

Examples of preferred cyclic peptides of the present invention, with an N-terminal hydrophobic modification (e.g. myristoylation) are:
Myr-cyclo [HBVpreS9-15] (SEQ. ID NO: 2) myr-cyclo [NPLGFFP]
Myr-cyclo [HBVpreS9-16] (SEQ. ID NO: 6) myr-cyclo [NPLGFFPD]
Myr-cyclo [HBVpreS8-16] (SEQ. ID NO: 9) myr-cyclo [PNPLGFFPD]
Myr-cyclo [HBVpreS9-17] (SEQ. ID NO: 12) myr-cyclo [NPLGFFPDH]
Myr-cyclo [HBVpreS8-17] (SEQ. ID NO: 15) myr-cyclo [PNPLGFFPDH]
Myr-cyclo-[HBVpreS2-21] (SEQ. ID NO: 18) myr-cyclo [GTNLSVPNPLGFFPDHQLDP]
Myr-cyclo-[HBVpreS2-48] (SEQ. ID NO: 21) myr-cyclo [GTNLSVPNPLGFFPDHQLDPAFGANSNNPDWDFNPNKDHWPEANQVG]
Myr-cyclo-[HBVpreS2-48] = cyclic Myrcludex B (SEQ. ID NO: 24) myr-cyclo [GTNLSVPNPLGFFPDHQLDPAFGANSNNPDWDFNPNKDHWPEANKVG]

In other embodiments, the cyclic peptides comprise P NPLGFXaaP with Xaa = L, i.e. NPLGFLP (SEQ ID NO. 3).

Respective examples of preferred cyclic peptides of the present invention are:
cyclo [NPLGFLP] (SEQ ID NO. 3)
cyclo [NPLGFLPD] (SEQ ID NO. 27)
cyclo [PNPLGFLPD] (SEQ ID NO. 28)
cyclo [NPLGFLPDH] (SEQ ID NO. 29)
cyclo[PNPLGFLPDH] (SEQ ID NO. 30)
cyclo[GTNLSVPNPLGFLPDHQLDP] (SEQ ID NO. 31)
cyclo [GTNLSVPNPLGFLPDHQLDPAFGANSNNPDWDFNPNKDHWPEANQVG] (SEQ ID NO. 32)
cyclo [GTNLSVPNPLGFLPDHQLDPAFGANSNNPDWDFNPNKDHWPEANKVG] (SEQ ID NO. 33).

As discussed above, the present invention provides a cyclic peptide having the general formula II

**Rₚ-(X)ₘ-P'-(Y)ₙ-(Z)ₒ-R_{q}** **(II)**

wherein
**P'** is the amino acid sequence NPLGFxₐPx_{b} (SEQ. ID NO: 34),
   **xₐ** is F or L or a natural or artificial amino acid with comparable physicochemical side chain properties,
   **x_{b}** is an amino acid with an acidic side chain, preferably D or E,
**X** is an amino acid sequence having a length of **m** amino acids,
   wherein **m** is 0 or 1;
**Y** is an amino sequence having a length of **n** amino acids,
   wherein **n** is 0, 1, 2 or at most 3;
   and wherein **m** + **n ≥** 0;
**Z** is an amino acid sequence of **o** amino acids,
   wherein **o** is 0 or 1,
   and which comprises a reactive side chain, e.g. amine, thiol, acidic side chain, hydroxyl, azid,
      such as aminoproline,
**Rₚ** is a hydrophobic moiety
   **p** is 0 or at least 1,
**R_{q}** is a hydrophobic moiety
   **q** is 0 or at least 1,
   and wherein **p** + **q** ≥ 1;
or a pharmaceutically acceptable salt thereof.

The cyclic peptides having the general formula II are conformationally constrained.

In one embodiment, the hydrophobic moiety **Rₚ** and/or the hydrophobic moiety **R_{q}** of the cyclic peptides having the general formula II is an acylation and/or addition of hydrophobic moieties,
preferably an acylation with C8 to C22 fatty acids (such as myristoyl (C 14), palmitoyl (C16) or stearoyl (C18), more preferably myristoyl (C14)),
or the hydrophobic moiety or moieties is selected from cholesterol, cholesterol derivatives, phospholipids, glycolipids, glycerol esters, steroids, ceramids, isoprene derivatives.

The cyclic peptides of the present invention having the general formula II can be cyclized in different ways, preferably
(a) via head-to-tail cyclization,
   from the N-terminus to the C-terminus and vice versa,
      - preferably via an amino modified carboxylic acid with variable distance between carboxylic and amino function,
         such as omega-aminohexadecanoic acid
      - such as via a lysine (K) at the N-terminus and the C-terminus (of the peptide sequence before cyclization),
(b) via a proline derivative,
   such as by using aminoproline at or near the N-terminus and a carboxylic acid containing side chain with or without a suitable linker at or near the C-terminus and vice versa (of the peptide sequence before cyclization),
(c) amide condensation of one amino acid side chain with another amino acid side chain or C-terminus or N-terminus (lactam),
   such as via amine containing side chain and a carboxylic acid side chain, for example a lysine (K) side chain at or near the N-terminus and an aspartic acid (D) side chain at or near the C-terminus and vice versa (of the peptide sequence before cyclization),
(d) macrolactonisation (e.g. Mukaiyama esterification),
   such as a serine (S) side chain with the acid side chain of aspartic acid (D),
(e) via thiol oxidation (disulfide bridge formation) of two thiol containing amino acids or artificial derivatives thereof comprised in the peptide,
   such as a cysteine (C) at or near the N-terminus and a cysteine (C) at or near the C-terminus (of the peptide sequence before cyclization),
(f) by formation of a diselenite or thioselenite,
   of either two selenol containing amino acids or one selenol containing amino acid and a thiol containing amino acid,
(g) via thioether formation,
   by reaction of a thiol containing amino acid with an amino acid comprising a suitable leaving group such as chloride,
(h) macrothiolactonisation of a suitable precursor,
   such as an aspartate beta-thioester with a lysine (K) attached 3-thiopropionate,
(i) ring closure olefm metathesis,
   such as of two allylglycine residues,
(j) thiazolin formation of suitable thiol and amine functions with an aldehyde moiety,
(k) triazole or tetrazole formation of an azide with an alkyne or cyanide moiety by copper mediated or copper free "click reactions",
(l) amination reactions e.g. reductive alkylation of an aldehyde with an amino function embedded in the peptide or Buchwald-Hartwig amination,
(m) C-C cross coupling reactions of suitable amino acid building blocks
   such as Prins, Heck or Hiyama coupling.

In one embodiment, the cyclic peptides of the present invention having the general formula II do not comprise peptides which are cyclized within the amino acid sequence of P' (i.e. within the pharmacophore of 7 amino acids, of SEQ ID NO. 34, i.e. via amino acid side chains of P'.

However, the P' sequence can be cyclized via head-to-tail cyclization.

In one embodiment, the cyclic peptides of the present invention having the general formula II comprise peptides which are cyclized within the amino acid sequence of P' (i.e. within the pharmacophore of 7 amino acids, of SEQ ID NO. 34, i.e. via amino acid side chains of P'.

In one embodiment, the cyclic peptides of the present invention having the general formula II further comprise depsipeptide bond(s) or functional group alkylations, such as backbone nitrogen methylations.

In a preferred embodiment **m** + **n** + **o** is 0 to 6,
and/or **m** = 0, 1 or 2 and/or **n** = 0 to 3, and/or **o**= 0 to 1.

In one embodiment, the cyclic peptide of the present invention having the general formula II comprises or consists of an amino acid sequence selected from the group of
- cyclo-intra[NP^{*}LGFFPD]
   having the linear amino acid sequence NPLGFFPD of SEQ ID NO. 6 and is cyclized between the second amino acid residue P ("P^{*}"), which is preferably an aminoproline, and the side chain of the C-terminal amino acid D 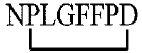 e.g. myr-cyclo-intra[NP^{*}LGFFPD]
- cyclo-intra[NCLGFFCD]y
   having the linear amino acid sequence NCLGFFCDy (SEQ ID NO. 35) and is cyclized between the two cysteines (i.e. the second amino acid residue, which is a cysteine, and a second cysteine) 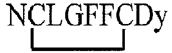
- cyclo[NPLGFFPDK]
   having the amino acid sequence NPLGFFPD-K (SEQ ID NO. 36) (which is preS9-16-Lys)
- cyclo[CNPLGFFPDCK]
   having the amino acid sequence C-NPLGFFPD-C-K (SEQ ID NO. 37) (which is Cys-preS9-16-Cys-Lys)
   and is cyclized between the two cysteines
- cyclo[KNPLGFFPDy]
   having the amino acid sequence K-NPLGFFPD-y (SEQ ID NO. 38) (which is Lys-preS9-16-(D-Tyr))
   and is preferably cyclized between the side chain of K and the C-terminal amino acid D-Tyr 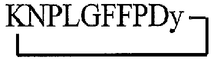
- *Compounds to be delivered*
The "compound" to be specifically delivered to the liver according to this invention can be any kind of compound.

In one embodiment, a cyclic peptide of the present invention having the general formula I or II comprises *compound(s).*

Such compound or compounds can be
- drug(s) or their respective prodrug(s);
- tag(s);
- label(s),
   such as fluorescent dye(s), radioisotope(s) and contrast agent(s);
- recombinant virus(s) or derivative(s) thereof;
- carrier or depot(s) for drug(s), prodrug(s) or label(s);
- immunogenic epitope(s);
- hormones;
- inhibitor(s), such as HBV capsid inhibitor;
- toxins
- or combinations thereof.

Such compound(s) are to be delivered to the liver.

Such compound(s) are preferably covalently attached, such as via a linker, spacer and/or anchor group(s),
e.g. a cleavable linker.

Preferred radioisotopes are ¹³¹I, ¹²⁵I, ^{99m}Tc, ¹⁸F, ⁶⁸Ga, ¹¹¹In, ⁹⁰Y, ¹⁷⁷LU.

In one embodiment, amino acid residue lysine can be attached/coupled to DOTA[⁶⁸Ga] (i.e. K(DOTA[⁶⁸Ga])_{.}

Preferred fluorescent dyes are Alexa dyes, derivatives of rhodamine and fluorescein, Cy-dyes.

In one embodiment, amino acid residue lysine can be attached/coupled to the fluorescent dye Atto 488 (i.e. K(Atto₄₈₈).

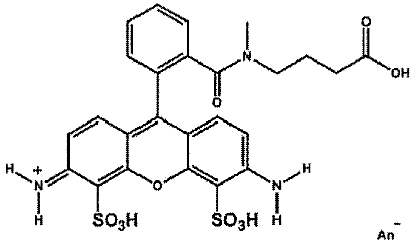

Preferred contrast agents are Gadolinium (Gd) complexes, supramagnetic iron (Fe) complexes and particles, compounds containing atoms of high atomic number, i.e. iodine for computer tomography (CT), microbubbles and carriers such as liposomes that contain these contrast agents.

In one embodiment, the compound is selected from a drug, preferably
interferon, such as IFNα;
viral reverse transcriptase inhibitor;
viral RNA polymerase inhibitor
viral core assembly inhibitor or viral nucleocapsid inhibitor;
kinase inhibitor, such as a Raf kinase inhibitor;
nucleoside analogue;
protease inhibitor;
proteasom inhibitor;
antibody or fragment thereof;
siRNA or precursor thereof;
farnesylation inhibitor; such as farnesyl transferase inhibitor (FTI);
alcohol dehydrogenase (ADH) or activator thereof;
cholesterol biosynthesis inhibitor;
inhibitor of the liver stage of a virus or a non-viral pathogen;
antibiotic.

In one embodiment, the drug is in form of a prodrug.

The envelope of HBV encloses three proteins termed L (large), M (middle) and S (small). They share the C-terminal S-domain with four transmembrane regions. The M- and L-protein carry additional N-terminal extensions of 55 and, genotype-dependent, 107 or 118 amino acids (preS2- and preS1).

Thus, the expression "preS-derived" peptide of HBV according to the present invention refers to a peptide with an amino acid sequence that corresponds to the N-terminal extensions of the L-protein of HBV, preS1, preferably to a consensus sequence of the species and genotypes A to H as well as of woolly monkey (WMHBV), chimpanzee and gorilla hepatitis B viruses, but it also refers to variants thereof, such as N-terminally and/or C-terminally truncated variants, amino acid substitution variants.

A peptide or amino acid sequence (a) preferably refers to a peptide with an amino acid sequence that corresponds to or is based on the N-terminal extensions of the L-protein of HBV, preS1, preferably of genotypes A to H as well as of woolly monkey (WMHBV), orangutan, chimpanzee and gorilla hepatitis B viruses and the recently described bat hepatitis B virus, but it also refers to variants thereof, preferably C-terminally truncated variants, amino acid substitution variants.

As an indispensible or essential sequence, the amino acid residues being important for the binding of the cyclic peptides of the present invention, as set out in P = SEQ ID NO: 1 (NPLGFXaaP) are present in the peptide/amino acid sequence of the cyclic peptides of the invention.

In particular, the peptides are based on the following sequences (amino acids in single letter code; essential domain or pharmacophore underlined).

Essential domain / pharmacopohore (SEQ ID NO: 1):
NPLGFXP (wherein X or Xaa is an arbitrary amino acid, preferably F or L, more preferably F)
preS HBV-A (ID: M57663; SEQ ID NO: 39): 
MGGWSSKPRKGMGTNLSVPNPLGFFPDHQLDPAFGANSNNPDWDFNPIKDHWPQANQVGVGAFGPGFTPPHGGVL

GWSPQAQGILATVPAMPPPASTNRQSGRQPTPISPPLRDSHPQA
preS HBV-B (ID: D00329, SEQ ID NO: 40) 
MGGWSSKPRKGMGTNLSVPNPLGFFPDHQLDPAFKANSENPDWDLNPHKDNWPDAHKVGVGAFGPGFTPPHGGLL

GWSPQAQGILTSVPAAPPPASTNRQSGRQPTPLSPPLRDTHPQA
preS HBV-C (ID: AB048704, SEQ ID NO: 41) 
MGGWSSKPRKGMGTNLSVPNPLGFFPDHQLDPAFKANSENPDWDLNPHKDNWPDAHKVGVGAFGPGFTPPHGGLL
GWSPQAQGILTSVPAAPPPASTNRQSGRQPTPLSPPLRDTHPQA
preS HBV-Chimpanzee (ID: AB032432, SEQ ID NO: 42) 
MGQNLSTSNPLGFFPEHQLDPAFKANTNNPDWDFNPKKDYWPEANKVGAGAFGPGFTPPHGGLLGWSPQAQGILT

TLPANPPPASTNRQSGRQPTPLSPPLRDTHPQA
preS HBV-D (ID: AB048702, SEQ ID NO: 43) 
MGQNLSTSNPLGFFPDHQLDPAFRANTNNPDWDFNPNKDTWPDANKVGAGAFGLGFTPPHGGLLGWSPQAQGFQT
LPANPPPASTNRQSGRQPTPLSPPLRTTHPQA
preS HBV-E (ID: X65657, SEQ ID NO: 44) 
MGLSWTVPLEWGKNISTTNPLGFFPDHQLDPAFRANTRNPDWDHNPNKDHWTEANKVGVGAFGPGFTPPHGGLLG

WSPQAQGMLKTLPADPPPASTNRQSGRQPTPITPPLRDTHPQA
preS HBV-F (ID: X69798@8, SEQ ID NO: 45) 
MGAPLSTTRRGMGQNLSVPNPLGFFPDHQLDPLFRANSSSPDWDFNTNKDSWPMANKVGVGGYGPGFTPPHGGLL
GWSPQAQGVLTTLPADPPPASTNRRSGRKPTPVSPPLRDTHPQA
preS HBV-G (ID: AF160501, SEQ ID NO: 46) 
MGLSWTVPLEWGKNLSASNPLGFLPDHQLDPAFRANTNNPDWDFNPKKDPWPEANKVGVGAYGPGFTPPHGGLLG

WSPQSQGTLTTLPADPPPASTNRQSGRQPTPISPPLRDSHPQA
HBV Gibbon (ID: AJ131572, SEQ ID NO: 47) 
MGQNHSVTNPLGFFPDHQLDPLFRANSNNPDWDFNPNKDTWPEATKVGVGAFGPGFTPPHGGLLGWSPQAQGILT

TLPAAPPPASTNRQSGRKATPISPPLRDTHPQA
HBV-H (ID: Q8JMY6, SEQ ID NO: 48) 
MGAPLSTARRGMGQNLSVPNPLGFFPDHQLDPLFRANSSSPDWDFNTNKDNWPMANKVGVGGFGPGFTPPHGGLL
GWSPQAQGILTTSPPDPPPASTNRRSGRKPTPVSPPLRDTHPQA
HBV Orangutan (ID: AF 193864, SEQ ID NO: 49) 
MGQNLSVSNPLGFFPEHQLDPLFRANTNNPDWDFNPNKDTWPEATKVGVGAFGPGFTPPHGGLLGWSPQAQGVTT

ILPAVPPPASTNRQSGRQPTPISPPLRDTHPQA
HBV Woolly Monkey (ID: NC 001896, SEQ ID NO: 50)

MGLNQSTFPLGFFPSHQLDPLFKANAGSADWDKPKDPWPQAHDTAVGAFGPGLVPPHGGLLGWSSQAQGLSVTVP

DTPPPPSTNRDKGRKPTPATPPLRDTHPQA

There also exists a HBV preS consensus sequence (for amino acid positions (-11) to 48) (SEQ ID NO: 51):

(-11)-M GGWSS TPRKG MGTNL SVPNP LGFFP DHQLD PAFRA NSNNP DWDFN PNKDH WPEAN
KVG-48

Furthermore, the peptide or amino acid sequences are preferably L-amino acid sequences, but can also comprise D-amino acids or are D-amino acid sequences.

Furthermore, the peptide or amino acid sequences can also comprise unnatural amino acids, which are preferably used for cyclization.

According to the invention, the cyclic peptides of the present invention with general formula I

**(X)ₘ-P-(Y)ₙ**

comprise or consist of at least the 7 amino acids having the sequence of SEQ ID NO: 1 (P).

Furthermore and discussed above, preferably, m + n is 0 to 42, and/or m = 0 to 8 and/or n = 0 to 34, provided that m + n is 0 or at least 1.

A cyclic peptide of the present invention with general formula I can comprise or consist of 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 amino acids.

According to the invention, the cyclic peptides of the present invention with general formula II

**Rₚ-(X)ₘ-P'-(Y)ₙ-(Z)ₒ-R_{q}**

comprise or consist of at least the 8 amino acids having the sequence of SEQ ID NO: 34 (P').

Furthermore and discussed above, preferably, m + n + o is 0 to 6,
and/or m = 0, 1 or 2 and/or n = 0 to 3, and/or o is 0 to 1.

A cyclic peptide of the present invention with general formula II can comprise or consist of 8, 9, 10, 11, 12, 13, 14 amino acids.

### - Synthesis of the cyclic peptides

The peptides of this invention can be prepared by a variety of procedures readily known to those skilled in the art, in general by synthetic chemical procedures.

Synthetic chemical procedures include more particularly the solid phase sequential and block synthesis (Erickson & Merrifield, 1976). The solid phase sequential procedure can be performed using established automated methods such as by use of an automated peptide synthesizer. In this procedure an α-amino protected amino acid is bound to a resin support. The resin support employed can be any suitable resin conventionally employed in the art for the solid phase preparation of (poly)peptides, preferably polystyrene which has been copolymerized with polyoxyethylen to provide sites for ester formation with the initially introduced o-amino protected amino acid. This optimized method, applied by the inventors, has been explicitly described (see e.g. Gausepohl *et al.,* 1989). The amino acids are introduced one by one (step-wise). Each synthesis cycle corresponding to the introduction of one amino acid includes a deprotection step, successive washing steps, a coupling step with activation of the amino acid, and subsequent washing steps. Each of these steps is followed by a filtration. The reactive agents for coupling are the classical reactive agents for (poly)peptide synthesis such as dicyclohexylcarbodiimide, hydroxybenzotriazole, benzotriazil-1-yl-oxytris (dimethylamino) phosphonium hexafluorophosphate, and diphenylphosphorylazide. After synthesis of the polypeptide on the resin, the polypeptide is separated from the resin by a treatment with a strong acid such as trifluoroacetic acid in the presence of anisol, ethanedithiol or 2-methylindole. The compound is then purified by the classical techniques of purification, in particular by means of HPLC.

The peptides of the present invention may also be obtained by coupling (poly)peptide fragments that are selectively protected, this coupling being effected e.g. in a solution.

For synthesis of Myrcludex B, see also Schieck *et al.,* 2010.

### Peptide cyclisation strategies

Generally, covalent peptide cyclisation can be achieved by the formation of any chemical bond. The main strategies followed are cyclisation by the formation of disulfide bonds or amide bonds. The general aspects of peptide cyclization have been comprehensively reviewed by White and Yudin (2011).

For peptide cyclisation, first the linear peptide is synthesized (in most cases by solid-phase peptide synthesis (SPPS)).
As described by White and Yudin (2011) amide bond formation - the head to tail cyclization requires the availability of a carboxylic acid moiety with an appropriate convergent protecting group, i.e. a carbamate linked to an allylic ester (an Alloc group). The cyclisation is achieved either on the solid support or in solution using activation agents such as HBTU. In case of peptides linked by disulfide bonds, the cyclization requires the availability of two thiol groups with an appropriate convergent protecting groups such as Trityl- protecting groups. The cyclisation is achieved by oxidation using agents such as hydrogen peroxide or milder conditions as available by air oxidation.

### Pharmaceutical compositions

As discussed above, the present invention provides a pharmaceutical composition.

The pharmaceutical compositions according to the present invention comprise
(i) at least one cyclic peptide of the present invention having general formula I and/or general formula II,
(ii) optionally, a pharmaceutically acceptable carrier and/or excipient.

The pharmaceutical compositions according to the present invention are very well suited for all the uses and methods described herein.

A "pharmaceutically acceptable carrier or excipient" refers to any vehicle wherein or with which the pharmaceutical or vaccine compositions according to the invention may be formulated. It includes a saline solution such as phosphate buffer saline. In general, a diluent or carrier is selected on the basis of the mode and route of administration, and standard pharmaceutical practice.

In a preferred embodiment, the pharmaceutical compositions according to the present invention are formulated for oral administration and, thus, comprise suitable pharmaceutically acceptable carrier(s) and/or excipient(s) for oral administration.

Examples for pharmaceutically acceptable carrier(s) and/or excipient(s) are also liposomes.

### Vectors and shuttles for liver targeting

As outlined above, the present invention provides the cyclic peptides of general formula I and II as vehicle or shuttle for the specific delivery of compound(s) to the liver.

"Vehicle" or "shuttle" for the specific delivery of a compound or compounds to the liver according to the present invention refers to the liver tropism or hepatotropism of the cyclic peptides of the present invention as found by the inventors and described herein, i.e. to their capacity to selectively accumulate in the liver, preferably to selectively accumulate at the plasma membrane of hepatocytes as well as to selectively enter into hepatocytes.

The invention is based on the finding of a highly specific liver accumulation and on the identification of the determinants of the liver tropism of HBV in the preS1 sequence of HBV by the inventors. Thus, the invention uses the knowledge about the determinants of the liver tropism for the design of universal vehicles or shuttles for specific liver targeting or delivery, respectively.

The cyclic peptides of the present invention are versatile vehicles or shuttles for specifically delivering compound(s) to the liver.

Preferably, the specific delivery of a compound to the liver is the specific delivery of the compound to hepatocytes.

Furthermore, the compound can specifically be delivered to hepatocytes *in vitro* as well as *in vivo.*

The compound is preferably specifically delivered to the liver of an animal, preferably mammal or human, or a bird.

### Medical applications

As discussed above, the present invention provides the cyclic peptide of the present invention having general formula II or the pharmaceutical composition of the present invention for use in medicine.

Thus, the cyclic peptide(s) of the present invention or the pharmaceutical composition(s) according to the invention are suitable and, thus, provided for the diagnosis, prevention and/or treatment of diseases.

As discussed above, the present invention provides the cyclic peptide of the present invention having general formula I as well as the cyclic peptide of the present invention having general formula II and the respective pharmaceutical compositions for use in liver targeting, such as for the specific delivery of compound(s) to the liver.

As discussed above, the present invention provides the cyclic peptide of the present invention having general formula I as well as the cyclic peptide of the present invention having general formula II and the respective pharmaceutical compositions for use in the diagnosis, prevention and/or treatment of a liver disease or condition.

In one embodiment, the liver disease or condition is selected from hepatitis, cirrhosis, haemochromatosis, preferably hepatitis caused by hepatitis A, B, C, D, E, F, G and H virus or concomitant hepatitis caused by viruses.

In one embodiment, the liver disease or disorder is a disease which involves a liver stadium of a virus or a non-viral pathogen, such as a tropical disease, malaria, schistosomiasis, leishmaniasis, Morbus Wilson.

In one embodiment, the liver disease or disorder is a liver tumor, preferably hepatocellular carcinoma (HCC).

In one embodiment, the liver disease or disorder is a post-transplantation complication after liver transplantation related to bile salt accumulation within the biliary pathway / a post-transplantation-related liver dysfunction.

In one embodiment, the liver disease or condition is related to sodium taurocholate cotransporter polypeptide (NTCP)-mediated transport of compounds into hepatocytes, or necessitates a delivery of a compound, such as a drug or label, to the site or location of the disease or condition,
and preferably is a liver involved metabolic disease selected from
intrahepatic cholestasis,
poisoning of the liver (by liver toxins) / hepatotoxicity,
drug-induced cholestatic liver disease,
hyperlipidemia,
posthepatic cholestasis,
metabolic syndrome,
non-alcoholic fatty liver disease (NAFLD),
glycogen storage diseases.

Preferably, the compounds which are transported into hepatocytes via NTCP are preferably bile acids, steroids, conjugated and non-conjugated thyroid hormones, liver toxins, compounds that are covalently bound to taurocholate, bromosulphophthalein, drugs.

The inventors have identified and described the sodium taurocholate cotransporter polypeptide (NTCP) as target and for use in the prevention and/or treatment of certain liver diseases or conditions, such as liver diseases or conditions that are related to NTCP-mediated transport of compounds (such as bile acids etc.) into hepatocytes, preferably liver involved metabolic diseases (e.g. intrahepatic cholestasis, poisoning of the liver (by liver toxins) / hepatotoxicity, drug-induced cholestatic liver disease, hyperlipidemia, etc.) and cardiovascular diseases. See WO 2014/072524 and PCT/EP2014/066262, which are enclosed herewith in their entirety.

A "liver involved metabolic disease" when used herein refers to metabolic disorders including visceral obesity, diabetes mellitus and dyslipidemia which are influenced by the liver metabolism of lipids and bile acids.

In general, "cholestasis" is a condition where bile constituents cannot be secreted from hepatocytes into the biliary tree or where bile cannot flow from the liver to the duodenum, resulting in hepatocyte bile acid accumulation within hepatocytes.

"Cholestasis" or "intrahepatic cholestasis" when used herein refers to intrahepatic toxic effects of hepatocyte bile acid accumulation related to an insufficient expression and/or activity of bile salt pumps (like BSEP or MRP) in the canalicular membrane.

"Posthepatic cholestasis" when used herein refers to a cholestatic liver disease due to obstruction of the large bile ducts.

"Poisoning of the liver" or "hepatotoxicity" or "toxic liver disease" when used herein refer to toxic effects of drugs independent of bile acid accumulation. These drugs penetrate the hepatocytes via the NTCP-mediated transport and cause several direct toxic effects, by damaging the mitochondria or by activating enzymes in the cytochrome P-450 system leading to oxidative stress.

"Drug-induced cholestatic liver disease" when used herein refers to inhibition of the export of bile acids from hepatocytes due to drug effects on bile salt export pump (BSEP).
Drug-induced cholestasis may be caused by several drugs which inhibit BSEP, such as rifampicin, cyclosporine A, rifamycin SV, bosentan, troglitazone, erythromycin estolate, and glibenclamide (Fattinger *et al.,* 2001; Funk *et al.,* 2001; Funk *et al.,* 2001; Stieger *et al.,* 2000; Dawson *et al.,* 2012; Morgan *et al.,* 2010; Ogimura *et al.,* 2011). BSEP is a member of the ATP-binding cassette (ABC) family of transporters (BSEP is also identified as ABCB11) and it is involved in the process of exporting bile acids out of hepatocytes, thus reducing their toxicity to these cells. The above mentioned drugs cause the toxic effects of excess bile acid accumulation because the excretion of bile acid via BSEP is disabled. Inhibition of NTCP-mediated bile acid uptake via the lipopeptide-based compound (such as MyrB) and NTCP counterbalances BSEP inhibition, and thereby prevents hepatotoxicity or is suitable for treatment and/or diagnosis.

"Hyperlipidemia" (or hyperlipoproteinemia, or hyperlipidemia) involves abnormally elevated levels of any or all lipids and/or lipoproteins in the blood.

Hyperlipidemias are divided in primary and secondary subtypes. Primary hyperlipidemia is usually due to genetic causes (such as a mutation in a receptor protein), while secondary hyperlipidemia arises due to other underlying causes such as diabetes. Lipid and lipoprotein abnormalities are common in the general population, and are regarded as a modifiable risk factor for cardiovascular disease due to their influence on atherosclerosis.

"Hypercholesterolemia" (or hypercholesterolaemia) is the presence of high levels of cholesterol in the blood. It is a form of "hyperlipidemia".

"Hyperlipidemia" when used herein preferably refers to hypercholesterolemia which includes elevated LDL cholesterol, reduced HDL cholesterol, elevated triglycerides, clogged arteries leading to high blood pressure, cardiovascular disease (CVD), heart attacks and strokes.

"Metabolic syndrome" refers to a disorder of energy utilization and storage, diagnosed by a co-occurrence of three out of five of the following medical conditions: abdominal (central) obesity, elevated blood pressure, elevated fasting plasma glucose, high serum triglycerides, and low high-density cholesterol (HDL) levels. Metabolic syndrome increases the risk of developing cardiovascular disease, particularly heart failure, and diabetes. Metabolic syndrome is also known as metabolic syndrome X, cardiometabolic syndrome, syndrome X, insulin resistance syndrome, Reaven's syndrome, and CHAOS.

"Non-alcoholic fatty liver disease" (NAFLD) refers to one cause of a fatty liver, occurring when fat is deposited (steatosis) in the liver not due to excessive alcohol use. It is related to insulin resistance and the metabolic syndrome. Non-alcoholic steatohepatitis (NASH) is the most extreme form of NAFLD, and is regarded as a major cause of cirrhosis of the liver of unknown cause.

As discussed above, the present invention provides the cyclic peptide of the present invention having general formula I as well as the cyclic peptide of the present invention having general formula II and the respective pharmaceutical compositions for use in the diagnosis, prevention and/or treatment of a cardiovascular disease (CVD).

Preferably the cardiovascular disease comprises the control or modification of the cholesterol level or cholesterol uptake,
wherein the cholesterol level or uptake is preferably controlled or modified by decreasing or blocking the NCTP-mediated bile salt transport (by the cyclic peptide).

The cholesterol level or uptake is controlled or modified by decreasing or blocking the NCTP-mediated bile salt transport by the cyclic peptide of the present invention.

Said uses comprises the control or modification of the cholesterol level or cholesterol uptake, wherein the cholesterol level or uptake is controlled or modified by decreasing or blocking the NCTP-mediated bile salt transport by the cyclic peptide of the present invention.

Cardiovascular diseases (CVD) are the major cause of morbidity and death in the western world. High levels of cholesterol have been associated with CVD as one of the risc factors. Of particular importance clinically is the abnormal deposition of cholesterol and cholesterol-rich lipoproteins in the coronary arteries. Such deposition, eventually leading to atherosclerosis, is the leading contributory factor in diseases of the coronary arteries. In this case the management of CVD is critical dependent on lipid-lowering therapies. Different classes of drugs are available for this purpose, such as statins, cholesterol absorption inhibitors, bile acid resins, fibrates and nicotinic acid that act by reducing the levels of cholesterol by distinct pathways (Schmitz & Langmann, 2006). These drugs have several side effects and depend on the relative levels of the metabolizing enzymes and transporters that act on cardiovascular drugs.

The main control of cholesterol metabolism is caused by bile acid as an important regulator of cholesterol homeostasis. The levels of bile acid and cholesterol are linked by the regulation of cholesterol metabolism and absorption. The synthesis of the bile acids is the major pathway of cholesterol catabolism in mammals, because the end products of cholesterol utilization are the bile acids. The major pathway for the synthesis of the bile acids is initiated via hydroxylation of cholesterol at the 7 position via the action of cholesterol 7α-hydroxylase (CYP7A1).
That means that the synthesis of bile acids is one of the predominant mechanisms for the excretion of excess cholesterol. Under physiological conditions this regulation is insufficient to compensate for an excess intake of cholesterol. However, if bile acid uptake into hepatocytes is blocked, the excretion of cholesterol in the form of bile acids will be sufficient to compensate for an excess dietary intake of cholesterol. Blocking bile acid uptake via the lipopeptide-based compound according to the invention and NTCP leads to intracellular deficiency of bile acid which is compensated by increased cholesterol metabolism and absorption.

Thus, according to the invention, the cyclic peptides of the present invention are suitable for lipid-lowering therapies to prevent CVD.

### Route of administration

Preferably, the route of administration of cyclic peptides or pharmaceutical compositions of the present invention is selected from oral, subcutaneous, intravenous, nasal, intramuscular, transdermal, inhalative, by suppository.

A preferred route of administration or application is orally.

A preferred embodiment for nasal administration or application is a nasal spray.

### Therapeutically effective amount

The cyclic peptides or the pharmaceutical compositions of the invention are provided such that they comprise a therapeutically effective amount of said cyclic peptide(s) or of said pharmaceutical composition(s).

A "therapeutically effective amount" of a cyclic peptide or a pharmaceutical composition of this invention refers to the amount that is sufficient to inhibit NTCP receptor function. Furthermore, said "therapeutically effective amount" depends on the respective application and desired outcome of inhibition, treatment or vaccination.

A preferred therapeutically effective amount is in the range of 10 µg to 1 mg per kg body weight, preferably 10 µg to 100 µg.

Preferably, the therapeutically effective amount is in the range of from about 0.01 mg to about 50 mg per patient and per day, preferably from about 1 mg to about 20 mg per patient per day or is applied to a patient in a dose ranging from 100 nmol per kg to 2µmol per kg per day / or is applied to a patient in a dose ranging from 10 pmol per kg to 20µmol per kg body weight.

In case of an IC₅₀ value of the cyclic peptide used of about 10 nM, a preferred therapeutically effective amount is about 100 µg per kg body weight or in the range of 1 to 5 mg per patient. The preferred therapeutically effective amount in the range of 1 to 5 mg per patient can be administered once a day or in other embodiments only once every 2-3 days.

The skilled artisan will be able to determine suitable therapeutically effective amounts.

### Method for the specific delivery of compound(s) to the liver

As discussed above, the present invention provides a method for the specific delivery of compound(s) to the liver.

Said method comprises the administration of (a therapeutically effective amount of)
- a cyclic peptide of the present invention having the general formula I, or
- a cyclic peptide of the present invention having the general formula II, or
- a pharmaceutical composition of the present invention.

The cyclic peptides of the present invention having the general formula I and/or II preferably carry

### Methods of diagnosis, prevention and/or treatment of diseases

As discussed above, the present invention provides a method for the diagnosis, prevention and/or treatment of a liver disease or condition.

Said method comprises the administration of a therapeutically effective amount of
- a cyclic peptide of the present invention having the general formula I, or
- a cyclic peptide of the present invention having the general formula II, or
- a pharmaceutical composition of the present invention.

In one embodiment, the liver disease or condition is selected from hepatitis, cirrhosis, haemochromatosis, preferably hepatitis caused by hepatitis A, B, C, D, E, F, G and H virus or concomitant hepatitis caused by viruses.

In one embodiment, the liver disease or disorder is a disease which involves a liver stadium of a virus or a non-viral pathogen, such as a tropical disease, malaria, schistosomiasis, leishmaniasis, Morbus Wilson.

In one embodiment, the liver disease or disorder is a liver tumor, preferably hepatocellular carcinoma (HCC).

In one embodiment, the liver disease or disorder is a post-transplantation complication after liver transplantation related to bile salt accumulation within the biliary pathway.

In one embodiment, the liver disease or condition is related to sodium taurocholate cotransporter polypeptide (NTCP)-mediated transport of compounds into hepatocytes,
or necessitates a delivery of a compound, such as a drug or label, to the site or location of the disease or condition,
and preferably is a liver involved metabolic disease selected from
intrahepatic cholestasis,
poisoning of the liver (by liver toxins) / hepatotoxicity,
drug-induced cholestatic liver disease,
hyperlipidemia,
posthepatic cholestasis,
metabolic syndrome,
non-alcoholic fatty liver disease (NAFLD),
glycogen storage diseases,
and wherein the compounds which are transported into hepatocytes via NTCP are preferably bile acids, steroids, conjugated and non-conjugated thyroid hormones, liver toxins, compounds that are covalently bound to taurocholate, bromosulphophthalein, drugs.

As discussed above, the present invention provides a method for the diagnosis, prevention and/or treatment of a cardiovascular disease (CVD).

Said method comprises the administration of a therapeutically effective amount of
- a cyclic peptide of the present invention having the general formula I, or
- a cyclic peptide of the present invention having the general formula II, or
- a pharmaceutical composition of the present invention.

Said method preferably comprises the control or modification of the cholesterol level or cholesterol uptake,
wherein the cholesterol level or uptake is preferably controlled or modified by decreasing or blocking the NCTP-mediated bile salt transport (by the cyclic peptide).

In the methods of the present invention, and as discussed above, the "therapeutically effective amount" depends on the respective application and desired outcome of inhibition, treatment or vaccination, as discussed above.

In one embodiment, and as discussed above, the therapeutically effective amount is preferably in the range of from about 0.01 mg to about 50 mg per patient, preferably from about 1 mg to about 20 mg per patient,
or wherein the cyclic peptide is preferably applied to a patient in a dose ranging from 10 pmol per kg to 20µmol per kg body weight.

In the methods of the present invention, and as discussed above, the route of administration is preferably selected from oral, subcutaneous, intravenous, nasal, intramuscular, transdermal, inhalative, by suppository.

The following examples and drawings illustrate the present invention without, however, limiting the same thereto.

The following examples and drawings illustrate the present invention without, however, limiting the same thereto.

### BRIEF DESCRIPTION OF THE DRAWINGS

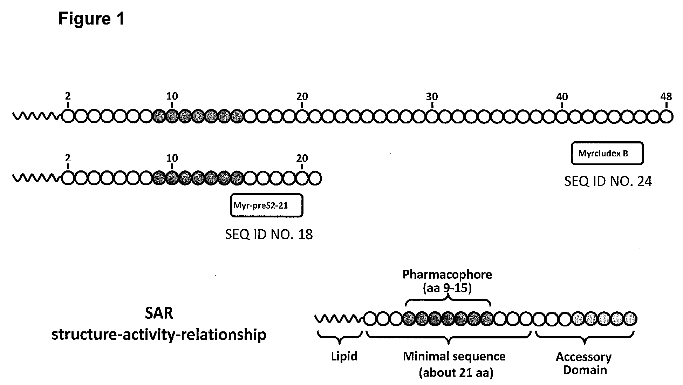

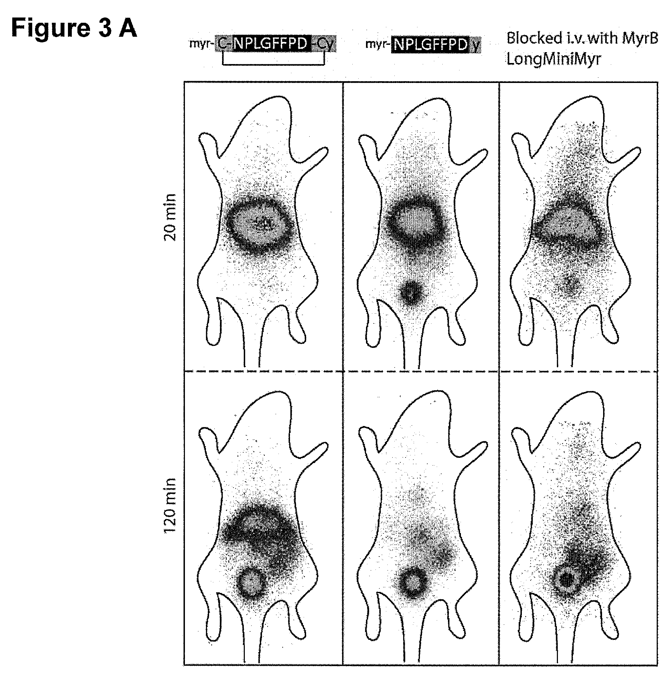

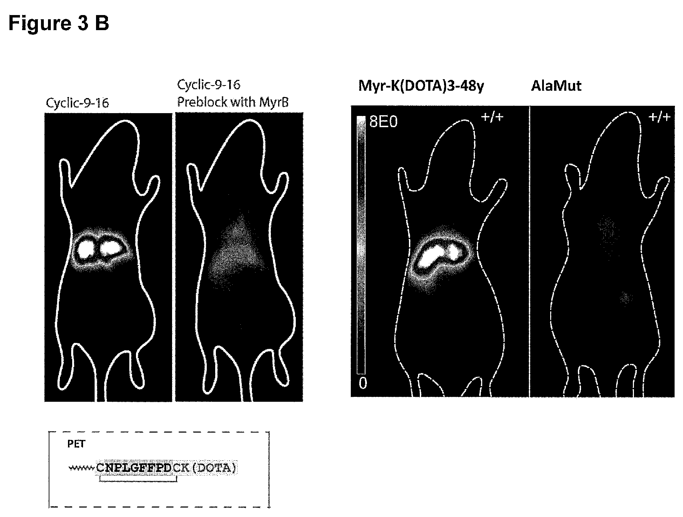

**Figure 1** *Myrcludex B and derivatives*

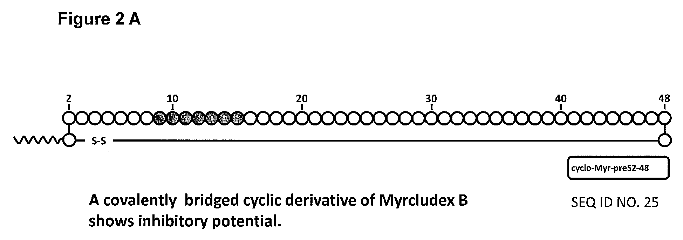

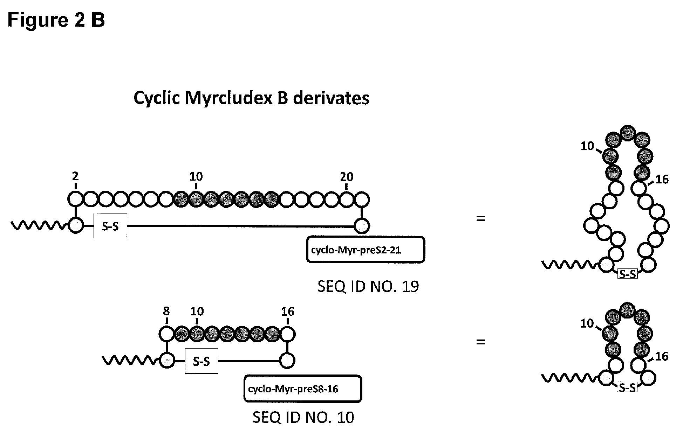

**Figure 2** *Cyclic derivatives of Myrcludex B.*
   (A) The sequence and structure of the covalently bridged cyclic derivative of Myrcludex B (Myr-2-48 Cyc).
   (B) Further cyclic Myrcludex B derivates
**Figure 3** *NTCP specific accumulation in the liver of cyclo[9-16]myr;*
   (A) Scintigraphic imaging of the distribution of the ¹²⁵I-labelled peptide myr-cyclo[CNPLGFFPDC]y and myr-NPLGFFPDy and the inhibitory effect of excess of Myrcludex B in healthy mice at 20 and 120 min p.i.
   (B) MicroPET of the ⁶⁸Ga-labeled cyclic DOTA conjugate "cyclic-9-16" and the ⁶⁸Ga-labeled DOTA conjugate "Myr-K(DOTA)3-48y" in healthy mice. The first two images show a comparison of the labeled "cyclic-9-16" with and without the addition of an excess of Myrcludex B. The third and the fourth image show the liver accumulation of the ⁶⁸Ga-labeled conjugate "Myr-K(DOTA)3-48y", a derivative without significant affinity to NTCP.

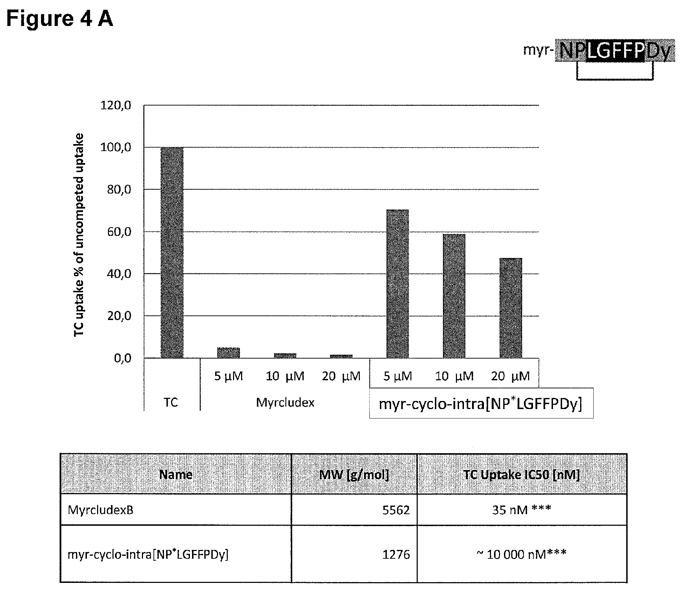

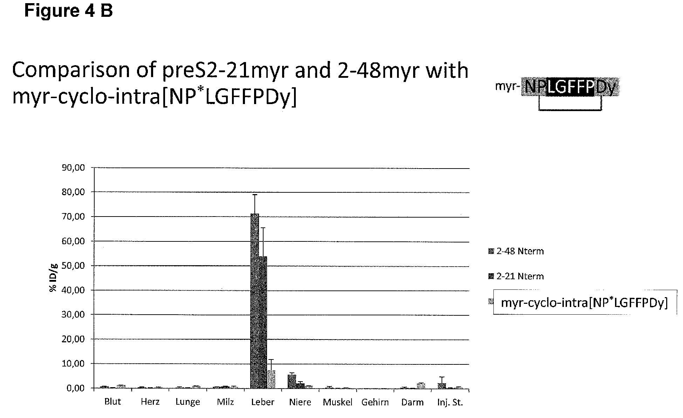

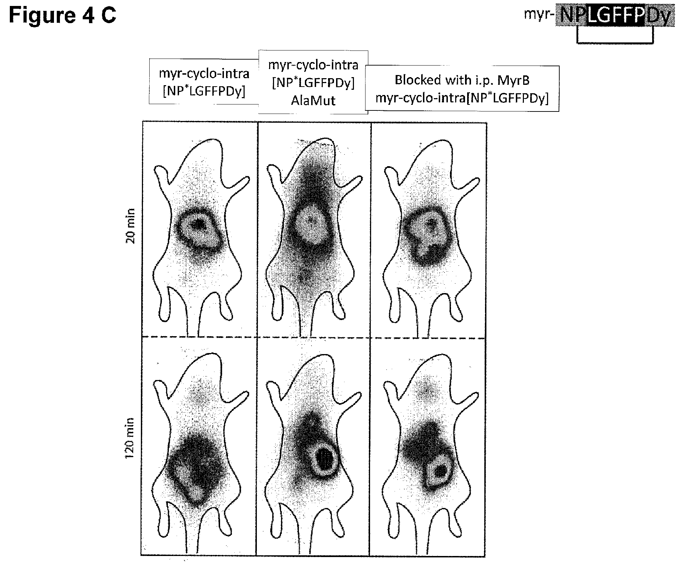

**Figure 4** *Liver targeting of the myr-cyclo-intra[NP^{*}GFFPDy] peptide*
   (A) TC uptake in differentiated HepaRG NTCP cells.
   (B) Biodistribution of the myr-cyclo-intra[NP^{*}LGFFPDy] in NMRI mice in comparison to the linear peptides preS2-21myr and 2-48myr.
   (C) y-camera with ¹²⁵I-labeled myr-cyclo-intra[NP^{*}LGFFPDy].

The peptide myr-cyclo-intra[NP^{*}LGFFPDy] accumulates rapidly in the liver and is eliminated via the intestine within the first 2 hours.

### EXAMPLES

### Example 1 Materials & Methods

### Abbreviations:

- COMU: (1-Cyano-2-ethoxy-2-oxoethylidenaminooxy)dimethylamino-morpholinocarbenium-hexafluorophosphate
- DCM: Dichloromethane
- DIPEA: *N*,*N*-diisopropylethylamine
- DMF: Dimethylformamide
- DOTA: 1,4,7,10-tetraazacyclododecane-1,4,7,10-tetraacetic acid
- Fmoc: Fluorenylmethyloxycarbonyl chloride
- Ga: Gallium
- HATU: 1-[Bis(dimethylamino)methylene]-1H-1,2,3-triazolo[4,5-b]pyridinium 3-oxid hexafluorophosphate
- HBeAg: Hepatitis B Virus Early Antigen
- HBcAg: Hepatitis B Core Antigen
- HBTU: 2-(1H-benzotriazol-1-yl)-1,1,3,3-tetramethyluronium hexafluorophosphate
- HBV: Hepatitis B Virus
- HPLC: High performance liquid chromatography
- LC/MS: Liquid chromatography-mass spectrometry
- NHS: *N*-Hydroxysuccinimide
- NMP: N-Methyl-2-pyrrolidone
- NTCP: Sodium taurocholate cotransporting polypeptide
- PBS: Phosphate buffered saline
- PET: Positron emission tomography
- PFA: Paraformaldehyde
- TC: Taurocholate
- TFA: Trifluoro acetic acid
- TIS: Triisopropylsilane

### 1. Peptide synthesis

### 1.1 Disulfide bridge peptide

### Solid phase peptide synthesis

Peptides were synthesized on solid phase (Tentagel R RAM resin, capacity 0.22 mmol/g, Rapp Polymere, Tubingen, Germany) using Fmoc/tBu chemistry in peptide synthesizer (Applied Biosystems 443A, Foster City, CA, USA). Before beginning peptide synthesis the resin (0.05 mmol) was preswollen in DCM. Fmoc-protected amino acids were used in a 10-fold excess (0.5mmol) and activated with HBTU/ DIPEA in NMP.
See also Schieck *et al.,* 2010.

### Myristoylation

Peptide on the solid support was swollen in DCM and washed with NMP. Myristic acid (4 eq.) and HATU or COMU (4 eq.) were dissolved in NMP and 10 eq. DIPEA were added. The mixture was added to the resin and was incubated for 30 min. Afterwards the resin was washed three times with NMP, three times with DCM and dried.

### Deprotection and cleavage from resin

The peptide was cleaved and deprotected with TFA/TIS/H₂O (95:2.5:2.5). The deprotected peptide was precipitated with diethyl ether, pelleted by centrifugation (3000 rpm, 5 min) and washed twice with fresh diethyl ether. The peptide was dried.
MyrB:
   Myr- GTNLSVPNPLGFFPDHQLDPAFGANSNNPDWDFNPNKDHWPEANKVG-amide [SEQ ID NO. 24]

### Disulfide bridge formation

5 mg of raw peptide were dissolved in 5 ml 80% acetic acid. 1mg of iodine in glacial acetic acid was slowly dropped into the peptide solution. 10 µl of saturated ascorbic acid solution were added. The solvent was evaporated and the peptide redissolved in 1:1 acetonitril: H₂O and purified with preparative HPLC. The success of the reaction was confirmed by LC/MS.

### Coupling of fluorescent dye/compounds

Peptides were dissolved in DMF and reacted with NHS-ester-activated compounds (2 eq.) and DIPEA (10 eq.). The reaction was controlled with HPLC.

### 1.2 Aminoproline-Peptide

200 mg (0.32 mmol) 2-Chlorotrityl chloride resin was charged with 41 mg (0.1 mmol) Fmoc-Glu(OAll)-OH and 52 mg (68 µL; 0.4 mmol) DIPEA in 2 mL Dichloromethane. After capping with methanol the resin was subjected to automated peptide synthesis (ABI 433A). 109 mg (0.25 mmol) Fmoc-L-Pro(4-NH-Alloc)-OH (2S,4S) were coupled at the desired amino acid position by COMU-activation. Solid phase cyclization was achieved by 110 mg (86 mL; 0.4 mmol) DPPA and 77 mg (102 µL; 0.6 mmol) in 2 mL NMP after linear assembly as well as catalytic allyl-deprotection with 5 mg tetrakis(triphenylphosphine)palladium(0) and 30 mg borane dimethylamine complex. The cyclic peptide was cleaved and deprotected by 95:2.5:2.5 TFA/water/TIS and purified by HPLC; occasionally a portion of the raw peptide was modified with DOTA or fluorescent dye active esters prior to purification

### 2. ⁶⁸Ga-Labeling and PET imaging

Ca. 1 mL of [⁶⁸Ga]Ga³⁺ eluate (ca. 600-800 MBq) was added to a mixture of 20 µL of a 1mM solution of compound xy in DMSO and 10 µL of saturated solution of ascorbic acid in water. The pH of the resulting mixture was adjusted to 3.5-4.0 by careful addition of a 2.5 M sodium acetate solution in water. Complexation was achieved by heating to over 95°C for 5-10 minutes under constant stirring. The product was isolated by solid phase extraction with ethanol followed by evaporation. The residue was taken up in 1% bovine serum albumin solution and an appropriate amount (ca. 20-50 MBq) was used for the individual experiment in a volume not exceeding 100 µl. Mice were anesthetized with 1% sevoflurane (Abbott, Wiesbaden, Germany) and images were recorded using an Inveon small animal positron emission tomographic (PET) scanner (Siemens, Knoxville, TN) up to 60 minutes postinjection.

### 3. ³H-taurocholate uptake assay

HepG2 NTCP cells seeded in a 24 well format were preincubated with the indicated peptide for 30 min at 37°C in culture medium. 150 µM taurocholate (containing 450 cpm/fmol ³H taurocholate) were added to each well and the cells were incubated an additional 15 minutes at 37°C. Uptake was stopped by removal of the cell culture medium and addition of ice cold PBS. The cells were washed three times with cold PBS and lysed (0.2 M NaOH, 0.05% SDS). Cell lysates were mixed with Ultima Gold liquid scintillation solution (Perkin Elmer, Rodgau, Germany) and the radioactivity measured in a liquid scintillation counter (Packard Instruments, Frankfurt, Germany).

The features disclosed in the foregoing description, in the claims and/or in the accompanying drawings may, both separately and in any combination thereof, be material for realizing the invention in diverse forms thereof.

### REFERENCES

Dawson S, et al. (2012) In vitro inhibition of the bile salt export pump correlates with risk of cholestatic drug-induced liver injury in humans. Drug Metab Dispos 40: 130-138.
Doring B, Lutteke T, Geyer J, Petzinger E. The SLC10 carrier family: transport functions and molecular structure. Curr Top Membr 2012;70:105-168.
Fattinger K, Funk C, Pantze M, et al. The endothelin antagonist bosentan inhibits the canalicular bile salt export pump: a potential mechanism for hepatic adverse reactions. Clin Pharmacol Ther. 2001; 69:223-31.
Funk C, Pantze M, Jehle L, et al. Troglitazone-induced intrahepatic cholestasis by an interference with the hepatobiliary export of bile acids in male and female rats. Correlation with the gender difference in troglitazone sulfate formation and the inhibition of the canalicular bile salt export pump (Bsep) by troglitazone and troglitazone sulfate. Toxicology. 2001; 167:83-98.
Funk C, Ponelle C, Scheuermann G, et al. Cholestatic potential of troglitazone as a possible factor contributing to troglitazone-induced hepatotoxicity: in vivo and in vitro interaction at the canalicular bile salt export pump (Bsep) in the rat. Mol Pharmacol. 2001; 59:627-35.
Gausepohl, H. et al. Int. J. Prot. Pept. Res. 34, 287-294 (1989).
Gripon P, Cannie I, Urban S. Efficient inhibition of hepatitis B virus infection by acylated peptides derived from the large viral surface protein. J Virol 2005;79:1613-1622.
Gripon, P., Le Seyec, J., Rumin, S. & Guguen-Guillouzo, C. Myristylation of the hepatitis B virus large surface protein is essential for viral infectivity. Virology 213, 292-299 (1995).
Kotani N, Maeda K, Debori Y, Camus S, Li R, Chesne C, Sugiyama Y. Expression and Transport Function of Drug Uptake Transporters in Differentiated HepaRG Cells. Mol Pharm 2012;9(12):3434-41.
Lempp FA, Urban S. Inhibitors of hepatitis B virus attachment and entry. Intervirology 2014;57:151-157.
Le Seyec, J., Chouteau, P., Cannie, I., Guguen-Guillouzo, C. & Gripon, P. Infection process of the hepatitis B virus depends on the presence of a defined sequence in the pre-S1 domain. J Virol 73, 2052-2057 (1999).
Meier A, Mehrle S, Weiss TS, Mier W, Urban S. The myristoylated preS1-domain of the Hepatitis B Virus L-protein mediates specific binding to differentiated hepatocytes. Hepatology 2012; 58:31-42.
Mitchell AR, Erickson BW, Ryabtsev MN, Hodges RS, and Merrifield RB, Tert-butoxycarbonylaminoacyl-4-(oxymethyl)-phenylacetamidomethyl-resin, a more acid-resistant support for solid-phase peptide synthesis. J Am Chem Soc. 1976;98(23):7357-62.
Morgan RE, et al. (2010) Interference with bile salt export pump function is a susceptibility factor for human liver injury in drug development. Toxicol Sci 118: 485-500.
Müller T, Mehrle S, Schieck A, Haberkorn U, Urban S, Mier W. Liver imaging with a novel hepatitis B surface protein derived SPECT-tracer. Mol Pharm. 2013;10(6):2230-6.
Nassal, M. Hepatitis B virus morphogenesis. Curr Top Microbiol Immunol 214, 297-337 (1996).
Ni Y, Lempp FA, Mehrle S, Nkongolo S, Kaufman C, Falth M, Stindt J, et al. Hepatitis B and D Viruses Exploit Sodium Taurocholate Co-transporting Polypeptide for Species-Specific Entry into Hepatocytes. Gastroenterology 2014;146:1070-1083.
Ogimura E, et al. (2011) Bile salt export pump inhibitors are associated with bile acid-dependent drug-induced toxicity in sandwich-cultured hepatocytes. Biochem Biophys Res Commun 416: 313-317.
Schieck A, Müller T, Schulze A, Haberkorn U, Urban S and Mier W. Solid-Phase Synthesis of the Lipopeptide Myr-HBVpreS/2-78, a Hepatitis B Virus Entry Inhibitor. Molecules 2010, 15(7), 4773-4783.
Schieck A, Schulze A, Gahler C, Muller T, Haberkorn U, Alexandrov A, Urban S, Mier W. Hepatitis B virus hepatotropism is mediated by specific receptor recognition in the liver and not restricted to susceptible hosts. Hepatology 2013; 58(1): 43-53. [Epub ahead of print: 2013. Jan 4.]
Schulze A, Schieck A, Ni Y, Mier W, Urban S. Fine mapping of pre-S sequence requirements for hepatitis B virus large envelope protein-mediated receptor interaction. J Virol 2010;84:1989-2000.
Seeger, C. & Mason, W.S. Hepatitis B virus biology. Microbiol Mol Biol Rev 64, 51-68 (2000).
Slijepcevic, D., Kaufman, C., Wichers, C.G.K., Gilglioni, E.H., Lempp, F.A., Duijst, S., de Waart, D.R., Oude Elferink, R.P.J., Mier, W., Stieger, B., Beuers, U., Urban, S., van de Graaf, S.F.J., 2015. Impaired uptake of conjugated bile acids and hepatitis b virus pres1-binding in na+-taurocholate cotransporting polypeptide knockout mice. Hepatology 62, 207-219
Stieger B, Fattinger K, Madon J, et al. Drug- and estrogen-induced cholestasis through inhibition of the hepatocellular bile salt export pump (Bsep) of rat liver. Gastroenterology. 2000; 118:422- 30.
Urban S, Future Virol. 2008, 3(3), 253-264.
Urban S, Bartenschlager R, Kubitz R, Zoulim F. Strategies to Inhibit Entry of HBV and HDV into Hepatocytes. Gastroenterology 2014;147(1):48-64.
White CJ and Yudin AK. Contemporary strategies for peptide macrocyclization, Nature Chemistry 2011; 3, 509-524.
Yan H, Zhong G, Xu G, He W, Jing Z, Gao Z, Huang Y, et al. Sodium taurocholate cotransporting polypeptide is a functional receptor for human hepatitis B and D virus. elife. 2012;1:e00049.

## Claims

1. A cyclic peptide of the general formula I
**(X)ₘ-P**-**(Y)ₙ** **(I)**
wherein
**P** is the amino acid sequence NPLGFXaaP (SEQ. ID NO: 1), with Xaa being F or L,
**X** is an amino acid sequence having a length of **m** amino acids,
wherein **m** is 0 or at least 1;
**Y** is an amino sequence having a length of n amino acids,
wherein **n** is 0 or at least 1;
and wherein **m** + **n** is 0 or at least 1;
or a pharmaceutically acceptable salt thereof,
for use in liver targeting.

2. The cyclic peptide for use according to claim 1, which is hydrophobically modified, wherein the hydrophobic modification(s) is/are preferably at amino acid side chain(s) of X and/or Y,
wherein the hydrophobic modification is preferably an acylation and/or addition of hydrophobic moieties,
more preferably an acylation with C8 to C22 fatty acids (such as myristoyl (C14), palmitoyl (C16) or stearoyl (C18), more preferably myristoyl (C14)),
or the hydrophobic moiety or moieties is preferably selected from cholesterol, cholesterol derivatives, phospholipids, glycolipids, glycerol esters, steroids, ceramids, isoprene derivatives.

3. The cyclic peptide for use according to claim 1 or 2, wherein the peptide is cyclized
(a) via thiol oxidation (disulfide bridge formation) of two cysteines (C) comprised in the peptide,
(b) amide condensation of two amino acid side chains (lactam),
(c) via head-to-tail cyclization,
(d) via backbone cyclization,
(e) via thioether formation,
and/or
(f) via hydrogen bond formation and/or bond-forming derivatives of amino acids.

4. The cyclic peptide for use according to any one of the preceding claims, wherein m + n is 0 to 42,
and/or wherein m = 0 to 8 and/or n = 0 to 34, provided that m + n is at least 1,
and/or having the general formula Ia
**cyclo[(X)ₘ-P-(Y)ₙ]** **(Ia)**
and carrying at least one hydrophobic modification at amino acid side chain(s) of X and/or P, such as
**H-cyclo[(X)ₘ-P-(Y)ₙ]**
wherein **H** is the hydrophobic modification.

5. The cyclic peptide for use according to any one of the preceding claims, wherein the peptide comprises or consists of an amino acid sequence selected from the group of
| | | | | |
|---|---|---|---|---|
| | HBVpreS9-15 | NPLGFFP | (SEQ ID NO. 2) | |
| | Cys-HBVpreS9-15-Cys | C-NPLGFFP-C | (SEQ ID NO. 4) | |
| | Cys-HBVpreS9-15-Cys-D-Tyr | C-NPLGFFP-C-y | (SEQ ID NO. 5) | |
| | HBVpreS9-16 | NPLGFFPD | (SEQ ID NO. 6) | |
| | Cys-HBVpreS9-16-Cys | C-NPLGFFPD-C | (SEQ ID NO. 7) | |
| | Cys-HBVpreS9-16-Cys-D-Tyr | C-NPLGFFPD-C-y | (SEQ ID NO. 8) | |
| | HBVpreS8-16 | PNPLGFFPD | (SEQ ID NO. 9) | |
| | Cys-HBVpreS8-16-Cys | | C-PNPLGFFPD-C | (SEQ ID NO. 10) |
| | Cys-HBVpreS8-16-Cys-D-Tyr | | C-PNPLGFFPD-C-y | (SEQ ID NO. 11) |
| | HBVpreS9-17 | | NPLGFFPDH | (SEQ ID NO. 12) |
| | Cys-HBVpreS9-17-Cys | | C-NPLGFFPDH-C | (SEQ ID NO. 13) |
| | Cys-HBVpreS9-17-Cys-D-Tyr | | C-NPLGFFPDH-C-y | (SEQ ID NO. 14) |
| | HBVpreS8-17 | | PNPLGFFPDH | (SEQ ID NO. 15) |
| | Cys-HBVpreS8-17-Cys | | C-PNPLGFFPDH-C | (SEQ ID NO. 16) |
| | Cys-HBVpreS8-17-Cys-D- Tyr | | C-PNPLGFFPDH-C-y | (SEQ ID NO. 17) |
| | HBVpreS2-21 | | GTNLSVPNPLGFFPDHQLDP | (SEQ ID NO. 18) |
| | Cys-HBVpreS2-21-Cys | | C-GTNLSVPNPLGFFPDHQLDP-C | |
| (SEQ ID NO. 19) | | | | |
| | Cys-HBVpreS2-21-Cys-D-Tyr | | C-GTNLSVPNPLGFFPDHQLDP-C-y | |
| (SEQ ID NO. 20) | | | | |
| | HBVpreS2-48 | GTNLSVPNPL GFFPDHQLDPAFGANSNNPDWDFNPNKDHWPEANQVG | | |
| (SEQ ID NO. 21) | | | | |
| | Cys-HBVpreS2-48-Cys | | | (SEQ ID NO. 22) |
| | C-GTNLSVPNPLGFFPDHQLDPAFGANSNNPDWDFNPNKDHWPEANQVG-C | | | |
| | Cys-HBVpreS2-48-Cys-D-Tyr | | | (SEQ ID NO. 23) |
| | C-GTNLSVPNPLGFFPDHQLDPAFGANSNNPDWDFNPNKDHWPEANQVG-C-y | | | |
| | Myrcludex B | GTNLSVPNPLGFFPDHQLDPAFGANSNNPDWDFNPNKDHWPEANKVG | | |
| (SEQ ID NO. 24) | | | | |
| | Cys- Myrcludex B-Cys | | | (SEQ ID NO. 25) |
| | C-GTNLSVPNPLGFFPDHQLDPAFGANSNNPDWDFNPNKDHWPEANKVG-C | | | |
| | Cys- Myrcludex B-Cys-D-Tyr | | | (SEQ ID NO. 26) |
| | C-GTNLSVPNPLGFFPDHQLDPAFGANSNNPDWDFNPNKDHWPEANKVG-C-y | | | |
such as
cyclo [NPLGFFP] (SEQ. ID NO: 2)
cyclo [NPLGFFPD] (SEQ. ID NO: 6)
cyclo [PNPLGFFPD] (SEQ. ID NO: 9)
cyclo [NPLGFFPDH] (SEQ. ID NO: 12)
cyclo [PNPLGFFPDH] (SEQ. ID NO: 15)
cyclo [GTNLSVPNPLGFFPDHQLDP] (SEQ. ID NO: 18)
cyclo [GTNLSVPNPLGFFPDHQLDPAFGANSNNPDWDFNPNKDHWPEANQVG] (SEQ. ID NO:21)
cyclo [GTNLSVPNPLGFFPDHQLDPAFGANSNNPDWDFNPNKDHWPEANKVG] (SEQ. ID NO: 24)
cyclo [NPLGFLP] (SEQ ID NO. 3)
cyclo [NPLGFLPD] (SEQ ID NO. 27)
cyclo [PNPLGFLPD] (SEQ ID NO. 28)
cyclo [NPLGFLPDH] (SEQ ID NO. 29)
cyclo [PNPLGFLPDH] (SEQ ID NO. 30)
cyclo [GTNLSVPNPLGFLPDHQLDP] (SEQ ID NO. 31)
cyclo[GTNLSVPNPLGFLPDHQLDPAFGANSNNPDWDFNPNKDHWPEANQVG] (SEQ ID NO. 32)
cyclo [GTNLSVPNPLGFLPDHQLDPAFGANSNNPDWDFNPNKDHWPEANKVG] (SEQ ID NO. 33).
e.g.
Myr-cyclo [HBVpreS9-15] (SEQ. ID NO: 2) myr-cyclo [NPLGFFP]
Myr-cyclo [HBVpreS9-16] (SEQ. ID NO: 6) myr-cyclo [NPLGFFPD]
Myr-cyclo [HBVpreS8-16] (SEQ. ID NO: 9) myr-cyclo [PNPLGFFPD]
Myr-cyclo [HBVpreS9-17] (SEQ. ID NO: 12) myr-cyclo [NPLGFFPDH]
Myr-cyclo [HBVpreS8-17] (SEQ. ID NO: 15) myr-cyclo [PNPLGFFPDH]
Myr-cyclo-[HBVpreS2-21] (SEQ. ID NO: 18) myr-cyclo [GTNLSVPNPLGFFPDHQLDP]
Myr-cyclo-[HBVpreS2-48] (SEQ. ID NO: 21) myr-cyclo [GTNLSVPNPLGFFPDHQLDPAFGANSNNPDWDFNPNKDHWPEANQVG]
Myr-cyclo-[HBVpreS2-48] = cyclic Myrcludex B (SEQ. ID NO: 24) myr-cyclo [GTNLSVPNPLGFFPDHQLDPAFGANSNNPDWDFNPNKDHWPEANKVG]
e.g.
myr-cyclo [NPLGFLP]
myr-cyclo [NPLGFLPD]
myr-cyclo [PNPLGFLPD]
myr-cyclo [NPLGFLPDH]
myr-cyclo [PNPLGFLPDH]
myr-cyclo [GTNLSVPNPLGFLPDHQLDP]
myr-cyclo [GTNLSVPNPLGFLPDHQLDPAFGANSNNPDWDFNPNKDHWPEANQVG]
myr-cyclo [GTNLSVPNPLGFLPDHQLDPAFGANSNNPDWDFNPNKDHWPEANKVG].

6. A cyclic peptide having the general formula II
**Rₚ-(X)ₘ-P'-(Y)ₙ**-**(Z)ₒ-R_{q}** **(II)**
wherein
**P'** is the amino acid sequence NPLGFxₐPx_{b} (SEQ. ID NO: 34),
**xₐ** is F or L or a natural or artificial amino acid with comparable physicochemical side chain properties,
**x_{b}** is an amino acid with an acidic side chain, preferably D or E,
X is an amino acid sequence having a length of m amino acids,
wherein **m** is 0 or 1;
**Y** is an amino sequence having a length of **n** amino acids,
wherein **n** is 0, 1, 2 or at most 3;
and wherein **m** + **n** ≥ 0;
**Z** is an amino acid sequence of **o** amino acids,
wherein **o** is 0 or 1,
and which comprises a reactive side chain, e.g. amine, thiol, acidic side chain, hydroxyl, azid,
such as aminoproline,
**Rₚ** is a hydrophobic moiety
**p** is 0 or at least 1,
**R_{q}** is a hydrophobic moiety
**q** is 0 or at least 1,
and wherein **p** + **q** ≥ 1;
or a pharmaceutically acceptable salt thereof.

7. The cyclic peptide of claim 6, wherein the hydrophobic moiety **Rₚ** and/or the hydrophobic moiety **R_{q}** is an acylation and/or addition of hydrophobic moieties,
preferably an acylation with C8 to C22 fatty acids (such as myristoyl (C14), palmitoyl (C16) or stearoyl (C18), more preferably myristoyl (C14)),
or the hydrophobic moiety or moieties is selected from cholesterol, cholesterol derivatives, phospholipids, glycolipids, glycerol esters, steroids, ceramids, isoprene derivatives,
and/or wherein the peptide is cyclized
(a) via head-to-tail cyclization,
(b) via a proline derivative,
(c) amide condensation of one amino acid side chain with another amino acid side chain or C-terminus or N-terminus (lactam),
(d) macrolactonisation (e.g. Mukaiyama esterification),
(e) via thiol oxidation (disulfide bridge formation) of two thiol containing amino acids or artificial derivatives thereof comprised in the peptide,
(f) by formation of a diselenite or thioselenite,
(g) via thioether formation,
(h) macrothiolactonisation of a suitable precursor,
(i) ring closure olefin metathesis,
(j) thiazolin formation of suitable thiol and amine functions with an aldehyde moiety,
(k) triazole or tetrazole formation of an azide with an alkyne or cyanide moiety by copper mediated or copper free "click reactions",
(l) amination reactions e.g. reductive alkylation of an aldehyde with an amino function embedded in the peptide or Buchwald-Hartwig amination,
(m) C-C cross coupling reactions of suitable amino acid building blocks,
and/or further comprising depsipeptide bond(s) or functional group alkylations, such as backbone nitrogen methylations,
and/or wherein, preferably, **m** + **n** + **o** is 0 to 6,
and/or wherein **m** = 0, 1 or 2 and/or **n** = 0 to 3, and/or **o**= 0 to 1.

8. The cyclic peptide for use according to any one of claims 1 to 5 or the cyclic peptide of claim 6 or 7, which is cyclized within the amino acid sequence of P or P', i.e. via amino acid side chains of the amino acid residues of P or P',
such as cyclo-intra[NP*GFFPD] (having the linear amino acid sequence of SEQ ID NO. 6 being cyclized between the second amino acid residue P and the side chain of C-terminal D).

9. The cyclic peptide for use according to any one of claims 1 to 5 or 8, or the cyclic peptide of any one of claims 6 to 8, comprising compound(s),
such as
drug(s) or their respective prodrug(s);
tag(s);
label(s), such as fluorescent dye(s), radioisotope(s) and contrast agent(s); recombinant virus(s) or derivative(s) thereof;
carrier or depot(s) for drug(s), prodrug(s) or label(s);
immunogenic epitope(s);
hormones,
inhibitor(s); such as a HBV capsid inhibitor,
toxins,
or combinations thereof.
preferably covalently attached, such as via a linker, spacer and/or anchor group(s), e.g. a cleavable linker.

10. Pharmaceutical composition comprising
(i) at least one cyclic peptide as defined in any of claims 1 to 5 and 8, and/or at least one one cyclic peptide of any one of claims 6 to 8,
(ii) optionally, a pharmaceutically acceptable carrier and/or excipient.

11. The cyclic peptide of any one of claims 6 to 8 or the pharmaceutical composition of claim 10 for use in medicine,
preferably for use in liver targeting,
such as for the specific delivery of compound(s) to the liver.

12. The cyclic peptide for use according to any one of claims 1 to 5 and 8 or the cyclic peptide or pharmaceutical composition for use according to claim 11, for use in the diagnosis, prevention and/or treatment of a liver disease or condition,
wherein preferably the liver disease or condition is selected from hepatitis, cirrhosis, haemochromatosis, preferably hepatitis caused by hepatitis A, B, C, D, E, F, G and H virus or concomitant hepatitis caused by viruses,
and/or wherein preferably the liver disease or disorder is a disease which involves a liver stadium of a virus or a non-viral pathogen, such as a tropical disease, malaria, schistosomiasis, leishmaniasis, Morbus Wilson,
and/or wherein preferably the liver disease or disorder is a liver tumor, preferably hepatocellular carcinoma (HCC),
and/or wherein preferably the liver disease or disorder is a post-transplantation complication after liver transplantation related to bile salt accumulation within the biliary pathway,
and/or wherein preferably the liver disease or condition is related to sodium taurocholate cotransporter polypeptide (NTCP)-mediated transport of compounds into hepatocytes,
or necessitates a delivery of a compound, such as a drug or label, to the site or location of the disease or condition,
and preferably is a liver involved metabolic disease selected from
intrahepatic cholestasis,
poisoning of the liver (by liver toxins) / hepatotoxicity,
drug-induced cholestatic liver disease,
hyperlipidemia,
posthepatic cholestasis,
metabolic syndrome,
non-alcoholic fatty liver disease (NAFLD),
glycogen storage diseases,
and wherein the compounds which are transported into hepatocytes via NTCP are preferably bile acids, steroids, conjugated and non-conjugated thyroid hormones, liver toxins, compounds that are covalently bound to taurocholate, bromosulphophthalein, drugs.

13. The cyclic peptide for use according to any one of claims 1 to 5 and 8 or the cyclic peptide of any one of claims 6 to 8 or the pharmaceutical composition of claim 10 for use in the diagnosis, prevention and/or treatment of a cardiovascular disease (CVD), preferably comprising the control or modification of the cholesterol level or cholesterol uptake,
wherein the cholesterol level or uptake is preferably controlled or modified by decreasing or blocking the NCTP-mediated bile salt transport (by the cyclic peptide).

14. The cyclic peptide or pharmaceutical composition for use according to any one of claims 11 to 13, wherein the cyclic peptide is administered in a therapeutically effective amount, preferably in the range of from about 0.01 mg to about 50 mg per patient, preferably from about 1 mg to about 20 mg per patient,
or is applied to a patient in a dose ranging from 10 pmol per kg to 20µmol per kg body weight.

15. The cyclic peptide or pharmaceutical composition for use according to any one of claims 11 to 14, wherein the route of administration is selected from oral, subcutaneous, intravenous, nasal, intramuscular, transdermal, inhalative, by suppository.

16. A method for the specific delivery of compound(s) to the liver,
comprising
the administration of (a therapeutically effective amount of) a cyclic peptide as defined in any one of claims 1 to 5 or 8 or a cyclic peptide of any one of claims 6 to 8 or a pharmaceutical composition of claim 10.
